(19) Europäisches Patentamt
European Patent Office
Office européen des brevets

(11) Numéro de publication : **0 133 097**
**B1**

(12) # FASCICULE DE BREVET EUROPÉEN

(45) Date de publication du fascicule du brevet : **04.03.87**

(51) Int. Cl.⁴ : **C 07 K 7/00**, A 61 K 37/02

(21) Numéro de dépôt : **84401478.7**

(22) Date de dépôt : **12.07.84**

(54) **Nouveaux dérivés de synergistines, leur préparation et les compositions pharmaceutiques qui les contiennent.**

(30) Priorité : **13.07.83 FR 8311705**

(43) Date de publication de la demande : **13.02.85 Bulletin 85/07**

(45) Mention de la délivrance du brevet : **04.03.87 Bulletin 87/10**

(84) Etats contractants désignés : **AT BE CH DE FR GB IT LI LU NL SE**

(56) Documents cités :
**US-A- 4 355 112**
**PROCEEDINGS - AN INTERNATIONAL CONFERENCE ON TRENDS IN ANTIBIOTIC RESEARCH - GENETICS, BIOSYNTHESES, ACTIONS & NEW SUBSTANCES, 14-15 juin 1982, Tokyo, pages 89-98, Japan Antibiotics Research Association, Tokyo, JP; M.-L. CAPMAU et al.: "Mechanism of action of the pristinamycins"**
**CHEMICAL ABSTRACTS, vol. 71, no. 11, 1969, page 201, no. 47912e, Columbus, Ohio, US; M. DELEPINE: "Pristinamycin; an antibiotic with two synergystic components"**
**BULLETIN DE LA SOCIETE CHIMIQUE DE FRANCE, 1968, pages 585-591, FR; J PREUD'HOMME et al.: "Pristinamycine isolement, caractérisation et identification des constituants"**

(73) Titulaire : **RHONE-POULENC SANTE**
**Les Miroirs 18 Avenue d'Alsace**
**F-92400 Courbevoie Cedex (FR)**

(72) Inventeur : **Corbet, Jean-Pierre**
**"Les Marronniers" Résidence "Charrière Blanche"**
**F-69140 Ecully (FR)**
Inventeur : **Cotrel, Claude**
**17A avenue du Docteur Arnold Netter**
**F-75012 Paris (FR)**
Inventeur : **Farge, Daniel**
**30 rue des Pins Sylvestres**
**F-94320 Thiais (FR)**
Inventeur : **Paris, Jean-Marc**
**8 rue des Acacias**
**F-77360 Vaires-sur-Marne (FR)**

(74) Mandataire : **Gaumont, Robert et al**
**RHONE-POULENC RECHERCHES Service Brevets Pharma 25, Quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 133 097 B1

Jouve, 18, rue St-Denis, 75001 Paris, France

**Description**

La pristinamycine et la virginiamycine sont des produits connus : J. Preud'homme et coll., Bull. Soc. Chim. Fr., *2*, 585-91 (1968).

La présente invention concerne de nouveaux dérivés de synergistines de formule générale :

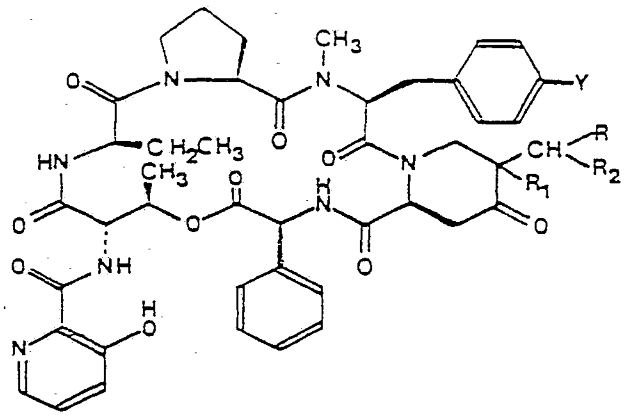

(I)

leurs sels, leur préparation et les compositions pharmaceutiques qui les contiennent.

Dans la formule générale (I), Y représente un atome d'hydrogène ou un radical diméthylamino et

a) soit $R_1$ et $R_2$ représentent chacun un atome d'hydrogène et R représente un radical pyrrolidinyl-3 thio ou pipéridyl-3 ou 4 thio (ces radicaux étant éventuellement substitués par un radical alcoyle) ou bien R représente un radical alcoylthio substitué par un ou deux radicaux hydroxysulfonyle, alcoylamino, dialcoylamino (éventuellement substitué par un radical mercapto ou dialcoylamino) ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle),

b) soit $R_1$ et $R_2$ forment ensemble une liaison de valence et R représente un radical pyrrolidinyl-3 amino, pipéridyl-3 ou 4 amino, pyrrolidinyl-3 oxy, pipéridyl-3 ou 4 oxy, pyrrolidinyl-3 thio, pipéridyl-3 ou 4 thio (ces radicaux étant éventuellement substitués sur l'atome d'azote du cycle par un radical alcoyle), ou bien R représente un radical alcoylamino, alcoyloxy ou alcoylthio substitués par un ou deux radicaux hydroxysulfonyle, alcoylamino, dialcoylamino (éventuellement substitué par un radical dialcoylamino, trialcoylammonio ou imidazolyle-4 ou 5 ou par un ou deux cycles choisis parmi pipérazino (éventuellement substitué par un radical alcoyle ou mercaptoalcoyle, morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle, étant entendu que, dans ce qui précède et ce qui suivra, les radicaux alcoyle et portions alcoyle contiennent, sauf mention spéciale, 1 à 5 atomes de carbone et sont en chaîne droite ou ramifiée.

Il est entendu que les produits de formule générale (I) pour lesquels $R_1$ et $R_2$ forment une liaison de valence peuvent se présenter sous 2 formes isomères et que ces isomères et leur mélange entrent dans le cadre de la présente invention.

A) Selon l'invention, les produits de formule générale (I) dans laquelle Y est défini comme précédemment et les autres symboles sont définis comme précédemment en a) peuvent être préparés par action d'un produit de formule générale :

$$R'—H \qquad (II)$$

dans laquelle R' a la définition de R donnée précédemment en a) sur un produit de formule générale :

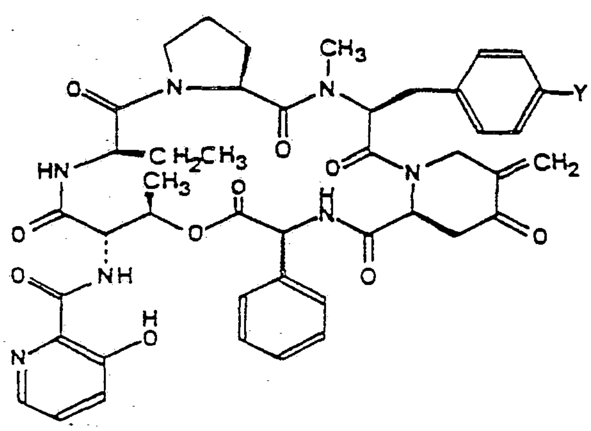

(III)

dans laquelle Y est défini comme précédemment.

On opère généralement dans un solvant organique tel qu'un alcool comme le méthanol ou un solvant chloré comme le chloroforme ou un mélange de ces solvants, à une température comprise entre 0 °C et la température de reflux du mélange réactionnel, de préférence à une température voisine de 20 °C.

Les produits de formule générale (III) peuvent être préparés par action d'un borohydrure alcalin en présence d'un acide organique fort sur un produit de formule générale :

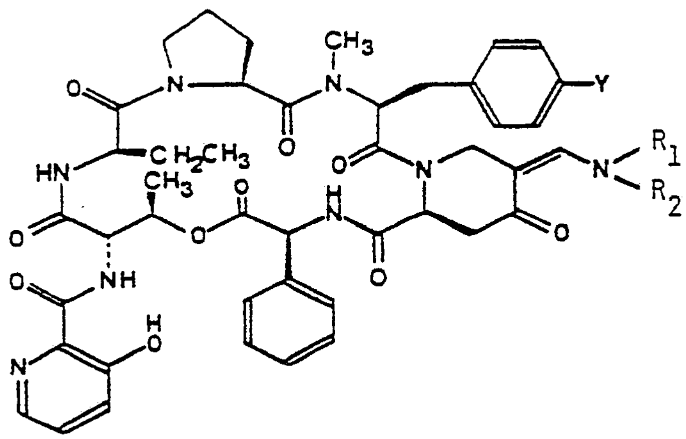

(IV)

dans laquelle Y est défini comme précédemment et $R_1$ et $R_2$ sont des radicaux alcoyle contenant 1 à 4 atomes de carbone ou forment ensemble avec l'atome d'azote auquel ils sont liés, un hétérocycle à 5 ou 6 chaînons contenant éventuellement un autre hétéroatome choisi parmi l'oxygène, l'azote ou le soufre.

On opère généralement au moyen de borohydrure ou de cyanoborohydrure de sodium dans un solvant organique tel qu'un éther comme le tétrahydrofuranne ou un alcool comme l'isopropanol, en présence d'un acide organique fort tel que l'acide trifluoroacétique, à une température comprise entre 0 °C et la température de reflux du mélange réactionnel, de préférence à une température voisine de 20 °C.

Les produits de formule générale (IV) peuvent être obtenus par action d'un produit de formule :

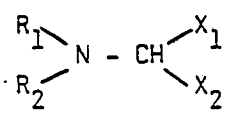

(V)

dans laquelle $R_1$ et $R_2$ sont définis comme précédemment et $X_1$ et $X_2$, identiques ou différents, représentent un radical alcoyloxy ou un radical amino substitué, défini comme $—NR_1R_2$ sur un produit de formule générale :

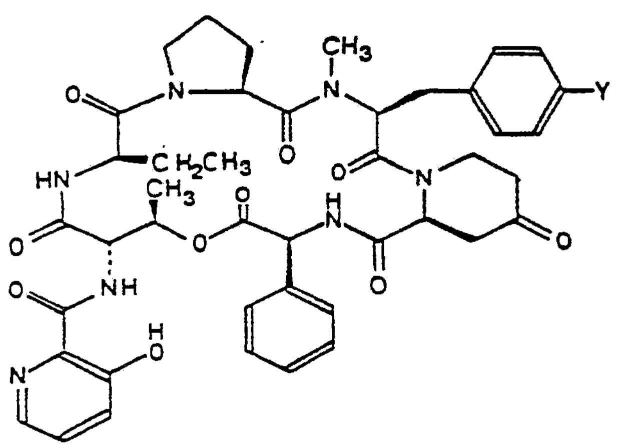

(VI)

dans laquelle Y est défini comme précédemment, c'est-à-dire la pristinamycine $I_A$ [$Y = N(CH_3)_2$] ou la virginiamycine S (Y = H).

Il est préférable d'utiliser un réactif de formule générale (V) dans laquelle $X_1$ et/ou $X_2$ sont choisis de telle manière que le radical amino substitué soit identique au groupement $—NR_1R_2$ présent sur la molécule.

Dans la pratique, il est avantageux de faire réagir le tert-butoxy bis(diméthylamino) méthane sur le produit de formule générale (VI) en opérant dans un solvant organique tel qu'un solvant chloré comme le dichloro-1,2 éthane ou un amide (diméthylformamide par exemple) à une température comprise entre 0 et 80 °C, de préférence à une température voisine de 20 °C.

Les produits de formule générale (V) peuvent être préparés selon les méthodes décrites par H. Bredereck et coll., Chem. Ber., *101*, 41 et 3058 (1968) et Chem. Ber., *106*, 3725 (1973).

B) Selon l'invention, les produits de formule générale (I) dans laquelle Y est défini comme précédemment et les autres symboles sont définis comme précédemment en b) à l'exception pour R de représenter un radical pyrrolidinyl-3 oxy, pipéridyl-3 ou 4 oxy ou alcoyloxy [éventuellement substitués comme défini en b)], peuvent être préparés par action d'un produit de formule générale :

$$R''—H \qquad (VII)$$

dans laquelle R″ a la définition de R donnée précédemment en b) à l'exception de représenter un radical pyrrolidinyl-3 oxy, pipéridyl-3 ou 4 oxy ou alcoyloxy [éventuellement substitué comme défini en b)], sur un produit de formule générale (IV) dans laquelle Y est défini comme précédemment et $R_1$ et $R_2$, identiques ou différents, représentent un atome d'hydrogène ou un radical phényle ou pyridyle éventuellement substitué (par un radical dialcoylamino dont la partie alcoyle contient 1 à 4 atomes de carbone en chaîne droite ou ramifiée) ou un radical alcoyle contenant 1 à 10 atomes de carbone en chaîne droite ou ramifiée éventuellement substitué [par un radical hydroxy, mercapto, carboxy, pyridyle, anilino, alcoylamino ou dialcoylamino dont au moins l'une des parties alcoyle est elle-même substituée par un radical hydroxy, mercapto, carboxy ou anilino], ou un radical alcényle contenant 3 ou 4 atomes de carbone, ou un radical alcynyle contenant 3 ou 4 atomes de carbone, ou bien $R_1$ et $R_2$ forment ensemble avec l'atome d'azote auquel ils sont liés un hétérocycle à 5 ou 6 chaînons contenant éventuellement un autre hétéroatome tel que l'oxygène, le soufre ou l'azote (éventuellement substitué par un radical alcoyle).

La réaction s'effectue en milieu organique, en présence d'un acide (par exemple l'acide acétique ou un mélange d'acide acétique et de quantités catalytiques d'acide trifluoracétique), en présence ou non d'un solvant, à une température comprise entre 0 et 50 °C. De préférence à une température voisine de 20 °C.

Le cas échéant, les solvants peuvent être choisis parmi les solvants organiques comme les éthers (tétrahydrofuranne), les alcools (éthanol) ou les solvants chlorés (chlorure de méthylène ou chloroforme par exemple).

Les produits de formule générale (IV) dans laquelle $R_1$ et $R_2$ sont définis comme précédemment à l'exception de prendre les significations définies pour la formule (IV) dans le procédé A), peuvent être obtenus par transénamination, par action d'une amine de formule $HNR_1R_2$ dans laquelle $R_1$ et $R_2$ sont définis comme ci-dessus sur une énamine de formule générale (IV) dans laquelle $R_1$ et $R_2$ sont définis comme précédemment dans le procédé A).

C) Selon l'invention, les produits de formule générale (I) dans laquelle Y est défini comme précédemment et les autres symboles sont définis comme précédemment en b) peuvent être préparés par action d'un produit de formule générale :

$$R'''—H \qquad (VIII)$$

dans laquelle R‴ est défini comme R en b), sur un produit de formule générale :

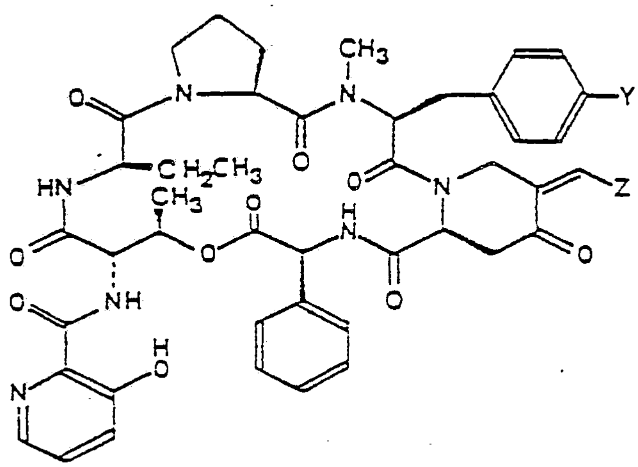

(IX)

dans laquelle Y est défini comme précédemment et Z représente un atome d'halogène, un radical triméthylsilyloxy, dialcoyloxyphosphoryloxy ou un radical de formule générale :

$$-OSO_2R_3 \qquad (IXa)$$

ou

$$-OCOR_4 \qquad (IXb)$$

dans lesquelles $R_3$ est un radical alcoyle, trifluorométhyle, trichlorométhyle ou un radical phényle éventuellement substitué par un atome d'halogène ou par un radical alcoyle ou nitro et $R_4$ est défini comme $R_3$ ou représente un radical alcoylcarbonylméthyle, alcoylcarbonyléthyle, alcoyloxycarbonylméthyle, alcoyloxycarbonyl-2 éthyle ou alcoyloxy.

Lorsque Z représente un atome d'halogène, il est choisi parmi le chlore et le brome.

On opère généralement dans un solvant organique tel qu'un éther comme le tétrahydrofuranne, un alcool comme l'éthanol ou un solvant chloré (chlorure de méthylène ou chloroforme par exemple) à une température voisine de 20 °C. La réaction s'effectue en milieu basique, par exemple en présence d'un hydrure alcalin ou d'un alcoolate alcalin (par exemple l'éthylate de sodium ou le tert-butylate de potassium).

Lorsque R''' est autre qu'alcoyloxy substitué ou hétérocyclyloxy, il est également possible d'opérer soit en milieu neutre, à une température comprise entre 0 et 50 °C, dans l'un des solvants cités ci-dessus, soit en milieu acide dans des conditions identiques à celles décrites précédemment pour le procédé B).

Les produits de formule générale (IX) peuvent être préparés par hydrolyse acide d'un produit de formule générale (IV) pour obtenir un produit de formule générale :

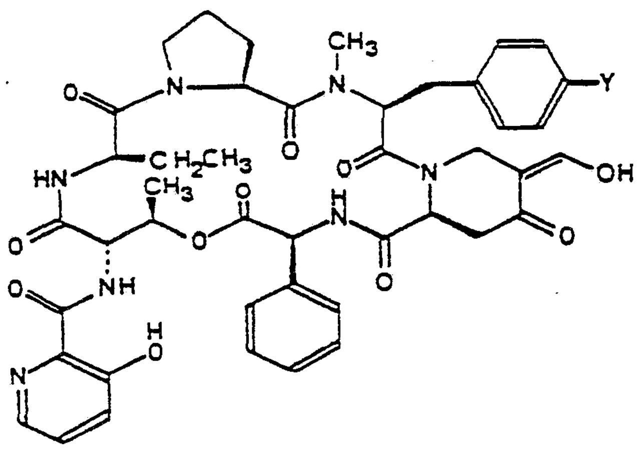

(X)

suivie :

α) ou bien de l'action d'un produit de formule générale :

$$Z'-X \qquad (XI)$$

dans laquelle X représente un atome d'halogène et Z' a la définition donnée précédemment pour Z à l'exception de représenter un atome d'halogène

β) ou bien de l'action d'un agent d'halogénation par exemple un produit de formule générale :

$$(C_6H_5)_3P(Hal)_2 \qquad (XII)$$

dans laquelle Hal représente un atome de chlore ou de brome, pour obtenir un produit de formule générale (IX) dans laquelle Z représente un atome d'halogène.

L'hydrolyse du produit de formule générale (IV) en produit de formule générale (X) s'effectue au moyen d'une solution aqueuse d'un acide minéral. On utilise par exemple une solution aqueuse 0,1N d'acide chlorhydrique et l'on opère à une température voisine de 20 °C.

La réaction du produit de formule générale (XI) sur le produit de formule générale (X) s'effectue généralement dans un solvant organique tel que le chlorure de méthylène en présence d'un accepteur d'acide tel qu'une base organique comme le triéthylamine ou une base minérale comme un carbonate ou un bicarbonate alcalin, par exemple le bicarbonate de sodium ou de potassium. On opère généralement à une température comprise entre —20 et + 20 °C.

La réaction du produit de formule générale (XII) sur le produit de formule générale (X) s'effectue généralement dans un solvant chloré tel que le chlorure de méthylène à une température comprise entre — 20 et + 20 °C.

Les produits de formules générales (II), (VII) et (VIII) peuvent être préparés selon ou par analogie avec les méthodes décrites ci-après dans les exemples et notamment selon :

— G. G. Urquhart et coll., Org. Synth., *21*, 36 (1941)
— A. I. Vogel, J. Chem. Soc., 1822 (1948)
— J. H. Chapman et L. N. Owen, J. Chem. Soc., 579 (1950)
— H. R. Snyder et coll., J. Am. Chem. Soc., *69*, 2672 (1947)
— D. D. Reynolds et coll., J. Org. Chem. *26*, 5125 (1961)

lorsqu'il s'agit d'un produit de formule générale (II), (VII) ou (VIII) pour lequel R', R'' ou R''' représente un radical alcoylthio substitué ou hétérocyclylthio, ou selon :

— A. J. W. Headlee et coll., J. Am. Chem. Soc., *55*, 1066 (1933)
— B. K. et K. N. Campbell, J. Am. Chem. Soc., *60*, 1372 (1938)
— R. C. Elderfield et coll., J. Am. Chem. Soc., *68*, 1579 (1946)

lorsqu'il s'agit d'un produit de formule générale (VII) ou (VIII) pour lequel R'' ou R''' représente un radical alcoyloxy substitué ou hétérocyclyloxy, ou selon :

— J. Amer. Chem. Soc., *54*, 1499 (1932) et J. Amer. Chem. Soc., *54*, 3441 (1932) lorsque R'' (ou R''') est un radical amino substitué
— E. F. Elslager et coll., J. Med. Chem., *17*, 99 (1974) et L. M. Werbel et coll., J. Het. Chem., *10*, 363 (1973) lorsque R'' (ou R''') est un radical hétérocyclylamino.

Il est entendu que, dans les méthodes ci-dessus, lorsque R', R'' ou R''' contiennent un radical alcoylamino pouvant interférer avec la réaction, ce dernier est préalablement protégé par toute méthode connue qui n'altère pas le reste de la molécule.

De même, lorsque les radicaux R', R'' et R''' dans les produits de formules générales (II), (VII) et (VIII) contiennent une fonction amine secondaire, il peut être éventuellement nécessaire de la protéger avant de faire réagir les produits correspondants respectivement sur les produits de formules générales (III), (IV) et (X). Le radical protecteur est éliminé après réaction. On utilise à cet effet tout moyen de blocage habituel employé pour protéger une fonction amine secondaire qui ne touche pas au reste de la molécule et qui s'élimine facilement. Il est particulièrement avantageux d'utiliser comme radication protecteur le radical trifluoroacétyle ; celui-ci peut ensuite être éliminé à l'aide d'une solution aqueuse de bicarbonate alcalin tel que le bicarbonate de sodium ou de potassium.

Il est également entendu que les produits de formules générales (IX) et (X) qui peuvent exister sous 2 formes isomères peuvent être employés indifféremment sous l'une ou l'autre de ces formes ou leur mélange.

Lorsque les produits de formule générale (I) présentent des formes isomères, il est possible de séparer celles-ci par toute méthode connue qui n'affecte pas le reste de la molécule ; par exemple par chromatographie liquide hautes performances.

Les nouveaux produits de formule générale (I) peuvent être purifiés par les méthodes connues habituelles telles que cristallisation, chromatographie ou extractions successives en milieu acide et basique. Pour l'homme du métier connaissant la sensibilité des synergistines en milieu alcalin, il est évident qu'on entend par « milieu basique » un milieu juste suffisamment alcalin pour libérer la substance-mère de son sel d'addition avec un acide, c'est-à-dire un milieu dont le pH n'excède pas 7,5 à 8.

Les nouveaux produits de formule générale (I) dans laquelle R représente un radical contenant une fonction aminée peuvent être transformés en sel d'addition avec les acides par action d'un acide dans un solvant organique tel qu'un alcool, une cétone, un ester ou un solvant chloré. Le sel précipite, éventuellement après concentration de sa solution ; il est séparé par filtration ou décantation. Les sels d'addition avec les acides peuvent également être obtenus à l'état de solutions aqueuses par addition d'une solution aqueuse d'acide correspondant sur le produit de formule générale (I).

Les nouveaux produits de formule générale (I) dans laquelle R représente un radical substitué par un ou deux radicaux hydroxysulfonyle peuvent être transformés en sel métallique ou en sels d'addition avec les bases azotées de manière analogue à celle décrite précédemment pour les sels d'addition avec les acides mais en remplaçant l'acide par un hydroxyde métallique ou une base azotée.

Il est bien connu que les synergistines obtenues par fermentation constituent des produits très recherchés par les médecins pour le traitement de beaucoup d'affections dues à des bactéries Gram-positives (du genre Staphylocoques, Streptocoques, pneumocoques, entérocoques) et Gram-négatives (du genre Haemophilus, gonocoques, méningocoques). Toutefois, ces produits présentent l'inconvénient d'être insolubles en milieu aqueux et ne peuvent donc être administrés que par voie orale, généralement sous forme de gélules, de dragées ou de comprimés. Compte tenu de cette insolubilité, il est impossible d'utiliser les synergistines connues lorsque le malade n'est pas en l'état de déglutir ; c'est notamment le cas en pédiatrie et en réanimation, alors que le spectre d'activité de ces produits en ferait une indication précieuse dans un grand nombre de circonstances, par exemple dans les cas de septicémies comateuses.

Les nouveaux produits selon l'invention présentent l'avantage considérable de pouvoir être solubilisés dans l'eau, à l'état de sels, aux doses thérapeutiquement utilisables, tout en conservant le spectre général d'activité des synergistines. Ils sont notamment actifs *in vitro* sur Staphylococcus aureus

Smith à des concentrations comprises entre 0,1 et 125 µg/ml.

Leur toxicité est généralement faible. Leur $DL_{50}$ est généralement supérieure à 300 mg/kg chez la souris par voie sous-cutanée.

Pour l'emploi thérapeutique, il peut être fait usage des nouveaux produits selon l'invention tels quels, c'est-à-dire à l'état de base, mais pour l'emploi en solution aqueuse, ce qui constitue l'avantage principal des produits selon l'invention, il est particulièrement avantageux de faire usage de leurs sels pharmaceutiquement acceptables, c'est-à-dire de sels non toxiques aux doses d'utilisation.

Comme sels pharmaceutiquement acceptables, on peut citer les sels d'addition avec les acides minéraux tels que chlorhydrates, bromhydrates, sulfates, nitrates, phosphates, ou organiques tels que acétates, propionates, succinates, maléates, fumarates, méthane-sulfonates, p-toluènesulfonates, iséthionates ou des dérivés de substitution de ces composés. Comme sels pharmaceutiquement acceptables, on peut encore citer les sels d'ammonium quaternaires lorsque R représente un radical trialcoylammonio ; ces sels correspondent aux anions des sels énumérés ci-dessus. Comme sels pharmaceutiquement acceptables, on peut encore citer les sels avec les métaux alcalins tels que les sels de sodium, de potassium, de lithium, avec les métaux alcalino-terreux tels que le sel de calcium ou de magnésium, le sel d'ammonium et les sels d'addition avec les bases organiques azotées : éthanolamine, diéthanolamine, triméthylamine, triéthylamine, méthylamine, propylamine, diisopropylamine, NN-diméthyléthanolamine, benzylamine, dibenzylamine, dicyclohexylbenzylamine, N-benzyl-β-phénéthylamine, NN'-dibenzyléthylènediamine, benzhydrylamine, arginine, leucine, lysine ou N-méthylglucamine.

D'un intérêt particulier sont les produits de formule générale (I) dans laquelle Y est un atome d'hydrogène ou un radical diméthylamino et

— soit $R_1$ et $R_2$ représentent chacun un atome d'hydrogène et R représente un radical pipéridyl-4 thio éventuellement substitué par un radical alcoyle, ou un radical alcoylthio substitué par un ou deux radicaux dialcoylamino (éventuellement substitué par un radical mercapto) alcoylpipérazino ou mercaptoalcoylpipérazino

— soit $R_1$ et $R_2$ forment ensemble une liaison de valence et R représente un radical pipéridyl-3 ou 4 amino ou pipéridyl-3 ou 4 thio éventuellement substitués sur l'atome d'azote du cycle par un radical alcoyle ou bien R représente des radicaux alcoylamino, alcoyloxy ou alcoylthio substitués par un ou deux radicaux hydroxysulfonyl, alcoylamino, dialcoylamino éventuellement substitué par un autre radical dialcoylamino, trialcoylammonio, imidazolyle-4 ou 5, alcoylpipérazino, morpholino, pipéridino, pyrrolidinyle ou N-alcoylpyrrolidinyle.

Parmi ces produits, plus spécialement actifs, les dérivés de synergistine de formule générale (I) dans laquelle Y est un atome d'hydrogène ou un radical diméthylamino et

— soit $R_1$ et $R_2$ représentent chacun un atome d'hydrogène et R représente un radical alcoylpipéridyl-4 thio ou un radical alcoylthio substitué par un ou deux radicaux dialcoylamino ou alcoylpipérazino,

— soit $R_1$ et $R_2$ forment ensemble une liaison de valence et R représente un radical alcoyl-1 pipéridyl-4 amino, ou bien R représente des radicaux alcoylamino en chaîne droite substitués par un radical alcoylamino ou dialcoylamino dont les parties alcoyle peuvent éventuellement former avec l'atome d'azote auquel elles sont rattachées, un hétérocycle choisi parmi pyrrolidinyl-1, pipéridino, alcoyl-4 pipérazino ou par un radical alcoyl-1 pyrrolidinyl-2, ou bien R représente un radical alcoylthio en chaîne droite substitué par un radical alcoyl-4 pipérazino ou représente un radical dialcoylamino-5 pentyle-2 ;

et notamment les produits de formule générale (I) pour lesquels Y est un atome d'hydrogène ou un radical diméthylamino et

— $R_1$ et $R_2$ représentent chacun un atome d'hydrogène et R représente un radical alcoylpipéridyl-4 thio ou un radical alcoylthio substitué par un ou deux radicaux dialcoylamino ou par un radical alcoylpipérazino, ou bien

— $R_1$ et $R_2$ forment ensemble une liaison de valence et R représente un radical alcoyl-1 pipéridyl-4 amino, étant entendu que les portions et radicaux alcoyle sont droits ou ramifiés et contiennent 1 à 3 atomes de carbone.

Et parmi ces produits les produits suivants :

— (diméthylamino-3 propyl) thiométhyl-5δ pristinamycine $I_A$,

— [(méthyl-4 pipérazinyl-1)-2 éthyl] thiométhyl-5δ pristinamycine $I_A$,

— [(méthyl-4 pipérazinyl-1)-3 propyl] thiométhyl-5δ pristinamycine $I_A$,

— [bis(diméthylamino)-1,3 propyl-2] thiométhyl-5δ pristinamycine $I_A$,

— (méthyl-1 pipéridyl-4) aminométhylène-5δ pristinamycine $I_A$.

Les exemples suivants, donnés à titre non limitatif, montrent comment l'invention peut être mise en pratique. Les spectres de RMN des produits illustrés dans ces exemples présentent des caractéristiques générales qui sont communes à tous les produits et des caractéristiques particulières qui sont propres à chacun des produits en fonction des substituants Y, $R_1$ et $R_2$. Dans l'exemple 1, il est donné l'attribution de tous les protons de la molécule ; dans les exemples suivants ne sont mentionnées que les caractéristiques particulières dues aux radicaux variables. Tous les protons sont désignés selon la numérotation indiquée dans la formule générale (XIII) et recommandée par J. O. Anteunis et al. [Eur. J. Biochim., *58*, 259 (1975)].

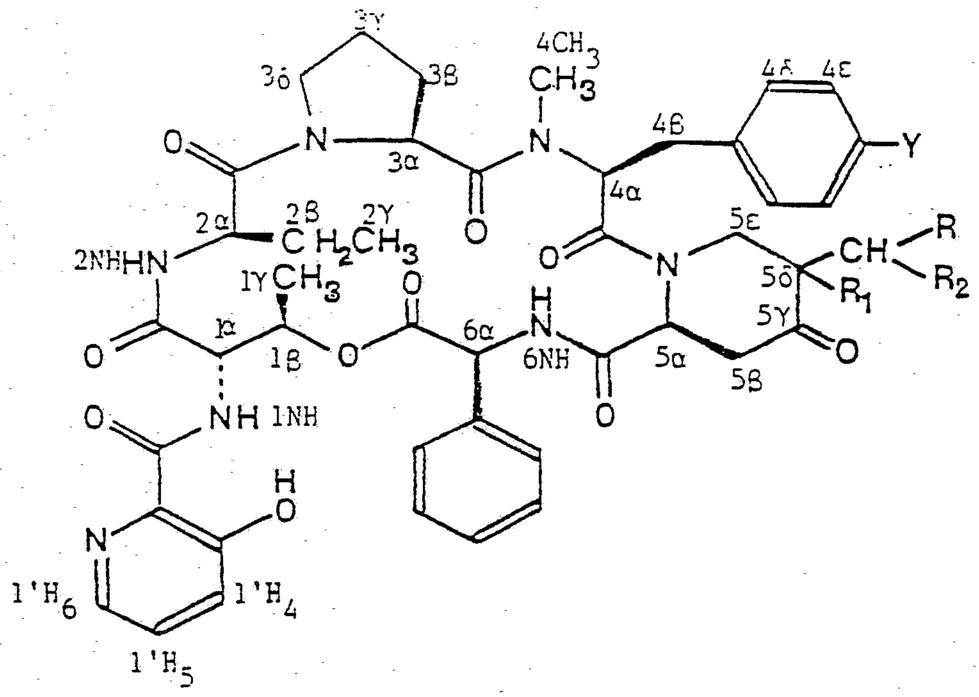

(XIII)

Tous les spectres ont été faits à 250 MHz dans le deutérochloroforme ; les déplacements chimiques sont exprimés en ppm par rapport au signal du tétraméthylsilane. Les abréviations utilisées dans la suite sont les suivantes :

s = singulet
d = doublet
t = triplet
mt = multiplet
m = massif
dd = doublet de doublet
dt = doublet de triplet
ddd = doublet de doublet de doublet
dddd = doublet de doublet de doublet de doublet

Dans les exemples 2 à 50 sont donnés entre parenthèses, respectivement : le déplacement chimique, la forme du signal, l'intégration (nombre de protons, avec éventuellement le pourcentage d'isomère) et l'attribution des protons.

Dans les exemples qui suivent, on appelle chromatographie « flash » une technique de purification caractérisée en ce qu'on utilise une colonne de chromatographie courte et qu'on opère sous une pression moyenne (50 kPa) en utilisant une silice de granulométrie 40-63 µm, selon W. C. Still, M. Kahn et A. Mitra [J. Org. Chem., *43*, 2923 (1978)].

Exemple 1

A une solution de 3,6 g de méthylène-5δ pristinamycine $I_A$ dans un mélange de 25 $cm^3$ de méthanol et de 5 $cm^3$ de chloroforme, on ajoute 1,95 g de diméthylamino-3 propanethiol, puis on agite la solution obtenue pendant 20 heures à une température voisine de 20 °C. Le mélange réactionnel est ensuite versé dans 250 $cm^3$ d'eau distillée ; l'émulsion obtenue est extraite 3 fois par 250 $cm^3$ au total de chlorure de méthylène. Les phases organiques sont réunies, séchées sur du sulfate de magnésium, filtrées puis concentrées à sec sous pression réduite (2,7 kPa) à 30 °C. Le résidu obtenu est purifié par chromatographie « flash » [éluant : chloroforme-méthanol (95,5 en volumes)] ; les fractions 10 à 38 sont concentrées à sec sous pression réduite (2,7 kPa) à 30 °C. Le résidu obtenu est trituré dans 30 $cm^3$ d'éther éthylique ; les cristaux obtenus sont séparés par filtration, puis séchés sous pression réduite (27 Pa) à 20 °C. On obtient ainsi 2,4 g de (diméthylamino-3 propyl) thiométhyl-5δ pristinamycine $I_A$ sous forme de cristaux blancs fondant à 234 °C.

(Voir tableau p. 9)

Spectre RMN :

| δ (ppm) | Forme | Attribution |
|---|---|---|
| 11,65 | s (large) | OH |
| 8,70 | d | 6 NH |
| 8,40 | d | 1 NH |
| 7,80 | dd | $1'H_6$ |
| 7,45 | m | $1'H_4$ + $1'H_5$ |
| 7,27 | m | } 6γ + 6δ + 6ε |
| 7,17 | m | |
| 7,05 | d } système AB | 4δ + 4ε |
| 6,60 | d | |
| 6,47 | d | 2 NH |
| 5,87 | ddd | 1β |
| 5,83 | d | 6α |
| 5,24 | m | 5α + 4α |
| 5,03 | ddd | $5\varepsilon_1$ |
| 4,85 | dd | 1α |
| 4,80 | m | 2α |
| 4,53 | dd | 3α |
| 3,53 | m | $3\delta_1$ |
| 3,35 | dd } système ABX | $-C\underline{H}_2-S-CH_2-$ |
| 3,15 | dd | |
| 3,25 | s | 4 $NCH_3$ |
| 3,25 | m | $3\delta_2$ |
| 2,90 | s | 4 $N(CH_3)_2$ |
| 2,90 | m | 4β |
| 2,55 | t | $-C\underline{H}_2N<^{CH_3}_{CH_3}$ |
| 2,50 | dd | $5\varepsilon_2$ |
| 2,40 | t | $-CH_2SC\underline{H}_2-$ |
| 2,40 à 2,20 | m | 5δ + $5\beta_1$ |
| 2,25 | s | $-CH_2N(C\underline{H}_3)_2$ |
| 2 | m | $3\beta_1$ |
| 1,75 | m | $-SCH_2C\underline{H}_2CH_2-$ |
| 1,8 à 1,45 | m | $2\beta_1$ + $2\beta_2$ + $3\gamma_1$ |
| 1,30 | d | 1γ |
| 1,25 à 1,05 | m | $3\gamma_2$ + $3\beta_2$ |

Tableau (Suite)

| δ (ppm) | Forme | Attribution |
|---|---|---|
| 0,9 | t | 2γ |
| 0,60 | dd | $5\beta_2$ |

On obtient une solution aqueuse à 10 % de (diméthylamino-3 propyl) thiométhyl-5δ pristinamycine $I_A$ (produit AA), avec :

| | |
|---|---|
| produit AA | 30 mg |
| acide chlorhydrique 0,1N | qsp 0,3 $cm^3$ |

La méthylène-5δ pristinamycine $I_A$ peut être préparée de la manière suivante :

A une solution de 12 g de diméthylaminométhylène-5δ pristinamycine $I_A$ dans 230 $cm^3$ de tétrahydrofuranne contenant 1,2 $cm^3$ d'acide trifluoroacétique, on ajoute 0,43 g de cyanoborohydrure de sodium. La solution obtenue est agitée pendant 4 heures à une température voisine de 20 °C, puis est concentrée à sec sous pression réduite (2,7 kPa) à 30 °C. Le résidu obtenu est purifié par chromatographie « flash » [éluant : chloroforme-méthanol (95,5 en volumes)] ; les fractions 4 à 15 sont concentrées à sec sous pression réduite (2,7 kPa) à 30 °C. On obtient ainsi 5,5 g de méthylène-5δ pristinamycine $I_A$ sous forme de cristaux blancs fondant à 245 °C.

Spectre RMN :

0,55 (dd, 1H : $5\beta_2$)
2,40 (d, 1H : $5\beta_1$)
3,55 (dd, 1H : $5\varepsilon_2$)
5,25 (m, 2H : $5\alpha + 5\varepsilon_1$)
5,30 et 6,10 (2s, 2H : $=C\langle^{\underline{H}}_{\underline{H}}$ )
7,85 (dd, 1H : $1'H_6$)

La diméthylaminométhylène-5δ pristinamycine $I_A$ peut être préparée de la manière suivante :

A une solution de 46 g de pristinamycine $I_A$ dans 460 $cm^3$ de dichloro-1,2 éthane, on ajoute 230 $cm^3$ de tert-butoxy bis(diméthylamino)méthane ; la solution obtenue est agitée pendant 18 heures à une température voisine de 20 °C. Le mélange réactionnel est dilué par 1 litre de chlorure de méthylène puis lavé 3 fois par 3 litres au total d'une solution aqueuse à 0,4 % de chlorure d'ammonium. La phase organique est séchée sur du sulfate de magnésium, filtrée puis concentrée à sec sous pression réduite (2,7 kPa) à 30 °C. Le résidu obtenu est trituré avec 600 $cm^3$ d'eau distillée ; le mélange est filtré et le produit solide est séché sous pression réduite (2,7 kPa) à 20 °C. On obtient 41 g de diméthylaminométhylène-5δ pristinamycine $I_A$ brute sous forme d'une poudre beige. Ce produit est d'une qualité suffisante pour être utilisé tel quel dans les phases ultérieures. Il peut toutefois être purifié de la manière suivante :

23,5 g de diméthylaminométhylène-5δ pristinamycine $I_A$ brute sont purifiés par chromatographie « flash » [éluant : chloroforme-méthanol (98-2 en volumes)]. Les fractions 16 à 25 sont réunies et concentrées à sec sous pression réduite (2,7 kPa) à 30 °C. On obtient ainsi 12 g de diméthylaminométhylène-5δ pristinamycine $I_A$ sous forme d'une poudre beige fondant vers 195 °C.

Spectre RMN :

0,9 (t, 3H : 2γ)
1,0 (dd, 1H : $5\beta_2$)
2,50 (d, 1H : $5\beta_1$)
3,10 (s, 6H : $-N(CH_3)_2$)
3,70 (d, 1H : $5\varepsilon_2$)
5,50 (d, 1H : $5\varepsilon_1$)
7,40 (s, 1H : $=C\underline{H}N(CH_3)_2$)
7,75 (dd, 1H : $1'H_6$)

Exemple 2

En opérant d'une manière analogue à celle décrite à l'exemple 1, mais à partir de 0,9 g de méthylène-5δ virginiamycine S et 0,52 g de diméthylamino-3 propanethiol et après purification par chromatographie « flash » [éluant : chloroforme-méthanol (90-10 en volumes)] et concentration à sec des fractions 13 à 25 sous pression réduite (2,7 kPa) à 30 °C, on obtient 0,3 g de (diméthylamino-3 propyl) thiométhyl-5δ virginiamycine S sous forme d'une poudre blanche fondant vers 142 °C.

Spectre RMN :

0,45 (dd, 1H : $5\beta_2$)

1,90 (m, 2H : $-SCH_2\underline{CH_2}CH_2N<$ )

2,40 (s, 6H : $-CH_2-N<\underline{CH_3}, \underline{CH_3}$)

2,60 (m, 4H : $-S-\underline{CH_2}-CH_2-\underline{CH_2}-N<$)

3,45 (d, 1H : $5\varepsilon_2$)

4,85 (m, 3H dont $5\varepsilon_1$)

5,25 (dd, 1H : 5α)

7,78 (dd, 1H : $1'H_6$)

On obtient une solution aqueuse à 10 % de (diméthylamino-3 propyl) thiométhyl-5δ virginiamycine S (produit AB), à l'état de chlorhydrate, avec :

| | |
|---|---|
| produit AB | 0,1 g |
| acide chlorhydrique | qsp 1 $cm^3$ |

La méthylène-5δ virginiamycine S peut être préparée d'une manière analogue à celle décrite à l'exemple 1 pour la méthylène-5δ pristinamycine $I_A$, mais à partir de 2 g de diméthylaminométhylène-5δ virginiamycine S et 74 mg de cyanoborohydrure de sodium. Après purification par chromatographie « flash » [éluant : chloroforme-méthanol (98-2 en volumes)] et concentration à sec des fractions 2 à 5 sous pression réduite (2,7 kPa) à 30 °C, on obtient 1 g de méthylène-5δ virginiamycine S sous forme d'une poudre beige fondant vers 190 °C.

Spectre RMN :

0,35 (dd, 1H : $5\beta_2$)

2,45 (dd, 1H : $5\beta_1$)

3,55 (dd, 1H : $5\varepsilon_2$)

5,25 (dd, 1H : $5\varepsilon_1$)

5,25 (m, 1H : 5α)

5,30 et 6,15 (2s, 2H : $=C<\underline{H}, \underline{H}$ )

7,75 (dd, 1H : $1'H_6$)

La diméthylaminométhylène-5δ virginiamycine S peut être obtenue en opérant d'une manière analogue à celle décrite à l'exemple 1 pour la diméthylaminométhylène-5δ pristinamycine $I_A$, mais à partir de 2 g de virginiamycine S et 10 $cm^3$ de bis-diméthylamino tert-butoxyméthane et après purification par chromatographie « flash » [éluant : chloroforme-méthanol (98-2 en volumes)] et concentration à sec des fractions 9 à 12 sous pression réduite (2,7 kPa) à 30 °C, on obtient 0,8 g de diméthylaminométhylène-5δ virginiamycine S sous forme d'une poudre jaune fondant vers 175 °C.

Spectre RMN :

0,9 (m, 4H : $2\gamma + 5\beta_2$)

3,05 (s, 6H : $=CH-N(\underline{CH_3})_2$)

3,65 (d, 1H : 5$\varepsilon_2$)
4,85 (d, 1H : 5$\varepsilon_1$)
5,15 (dd, 1H : 5α)
7,10 à 7,40 (m : aromatiques + =CH-N<)
7,70 (dd, 1H : 1'$H_6$)

## Exemple 3

En opérant d'une manière analogue à celle décrite à l'exemple 1, mais à partir de 6 g de méthylène-5δ pristinamycine $I_A$ et 4 cm³ de (méthyl-4 pipérazinyl-1)-2 éthanethiol et après purification par chromatographie « flash » [éluant : chloroforme-méthanol (97-3 en volumes)] et concentration à sec des fractions 8 à 20 sous pression réduite (2,7 kPa) à 30 °C, on obtient 2,6 g de [(méthyl-4 pipérazinyl-1)-2 éthyl] thiométhyl-5δ pristinamycine $I_A$ sous forme de cristaux blancs fondant à 216 °C.

Spectre RMN :

0,60 (dd, 1H : 5$\beta_2$)
2,27 (s, 3H : >N-$CH_3$)
2,40 à 2,80 (m, 11H : 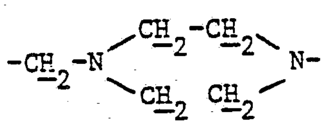 + 5$\beta_1$)
5,05 (dd, 1H : 5$\varepsilon_1$)
5,27 (m, 2H : 5α + 4α)
7,85 (mt, 1H x 0,8 : 1'$H_6$ 1er isomère)
7,95 (mt, 1H x 0,2 : 1'$H_6$ 2ème isomère)

On obtient une solution aqueuse à 5 % de [(méthyl-4 pipérazinyl-1)-2 éthyl] thiométhyl-5δ pristinamycine $I_A$ (produit AC, à l'état de chlorhydrate, avec :

| | |
|---|---|
| produit AC | 0,1 g |
| acide chlorhydrique 0,1N | 0,96 cm³ |
| eau distillée | qsp 2 cm³ |

Le (méthyl-4 pipérazinyl-1)-2 éthanethiol peut être préparé selon la méthode décrite par D. D. Reynolds et coll., J. Org. Chem. *26*, 5125 (1961).

## Exemple 4

En opérant d'une manière analogue à celle décrite à l'exemple 1, mais à partir de 2 g de méthylène-5δ pristinamycine $I_A$ et 3 cm³ de (méthyl-4 pipérazinyl-1)-3 propanethiol et après purification par chromatographie « flash » [éluant : chloroforme-méthanol (90-10 en volumes)] et concentration à sec des fractions 10 à 25 sous pression réduite (2,7 kPa) à 30 °C, on obtient 1,9 g de [(méthyl-4 pipérazinyl-1)-3 propyl] thiométhyl-5δ pristinamycine $I_A$ sous forme d'une poudre blanche fondant vers 156 °C.

Spectre RMN :

0,65 (dd, 1H : 5$\beta_2$)
2,30 (s, 3H : >N-$CH_3$)
2,50 (m, 13H : 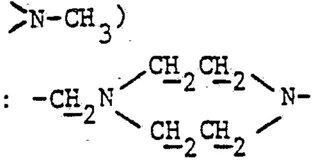 + -S$CH_2$- + 5$\beta_1$)
5,27 (m, 2H : 5α + 4α)
7,85 (dd, 1H x 0,8 : 1'$H_6$ 1er isomère)
7,95 (dd, 1H x 0,2 : 1'$H_6$ 2ème isomère)

On obtient une solution aqueuse à 10 % de [(méthyl-4 pipérazinyl-1)-3 propyl] thiométhyl-5δ pristinamycine $I_A$ (produit AD), à l'état de chlorhydrate, avec :

| | |
|---|---|
| produit AD | 0,1 g |
| acide chlorhydrique 0,5N | 0,38 $cm^3$ |
| eau distillée | qsp 1 $cm^3$ |

Le (méthyl-4 pipérazinyl-1)-3 propanethiol peut être préparé comme ci-après à l'exemple 41.

## Exemple 5

En opérant de manière analogue à celle décrite à l'exemple 1, mais à partir de 4 g de méthylène-5δ pristinamycine $I_A$ et 4 $cm^3$ de bis diméthylamino-1,3 propanethiol-2 et après purification par chromatographie « flash » [éluant : chloroforme-méthanol (95-5 en volumes)] et concentration à sec des fractions 20 à 60 sous pression réduite (2,7 kPa) à 30 °C, on obtient 0,59 g de [bis(diméthylamino)-1,3 propyl-2] thiométhyl-5δ pristinamycine $I_A$ sous forme d'une poudre blanche fondant vers 170 °C.

Spectre RMN :

0,63 (dd, 1H : $5\beta_2$)

2,40 (s, 6H : $-N(CH_3)_2$)

2,50 (m, 10H : 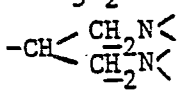 + $-N(CH_3)_2$)

4,97 (s, 1H : $5\varepsilon_1$)

5,30 (m, 2H : $5\alpha$ + $4\alpha$)

7,85 (mt, 1H x 0,85 : $1'H_6$ 1er isomère)

7,95 (mt, 1H x 0,15 : $1'H_6$ 2ème isomère)

On obtient une solution aqueuse à 7,5 % de bis[(diméthylamino)-1,3 propyl-2] thiométhyl-5δ pristinamycine $I_A$ (produit AE), à l'état de chlorhydrate, avec :

| | |
|---|---|
| produit AE | 0,03 g |
| acide chlorhydrique 0,1N | 0,3 $cm^3$ |
| eau distillée | qsp 0,4 $cm^3$ |

Le bis diméthylamino-1,3 propanethiol-2 peut être préparé comme ci-après à l'exemple 39.

## Exemple 6

En opérant d'une manière analogue à celle décrite à l'exemple 1, mais à partir de 3 g de méthylène-5δ pristinamycine $I_A$ et 0,97 g de méthyl-1 mercapto-4 pipéridine et après purification par chromatographie « flash » [éluant : chloroforme-méthanol (95-5 en volumes)] et concentration à sec des fractions 10 à 16 sous pression réduite (2,7 kPa) à 30 °C, on obtient 1,1 g de (méthyl-1 pipéridyl-4) thiométhyl-5δ pristinamycine $I_A$ sous forme d'une poudre blanche fondant à 260 °C.

Spectre RMN :

0,6 (dd, 1H : $5\beta_2$)

2 (m, 4H : -S- 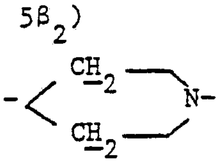 )

2,20 (s, 3H :—S— 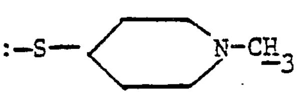 )

2,35 (m, 1H : $5\beta_1$)

2,90 (m, 4H : -S- 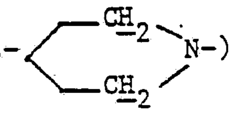

5,30 (m, 2H : 5α + 4α)

7,85 (dd, 1H : 1'$H_6$)

On obtient une solution aqueuse à 5 % de (méthyl-1 pipéridyl-4) thiométhyl-5δ pristinamycine $I_A$ (produit AF), à l'état de chlorhydrate, avec :

| | |
|---|---|
| produit AF | 0,03 g |
| acide chlorhydrique 0,1N | 0,3 $cm^3$ |
| eau distillée | qsp 0,6 $cm^3$ |

La méthyl-1 mercapto-4 pipéridine peut être préparée selon la méthode décrite par H. Barrer et R. E. Lyle, J. Org. Chem. *27,* 641 (1962).

Exemple 7

En opérant comme à l'exemple 1 mais à partir de 2 g de méthylène-5δ pristinamycine $I_A$ et de 0,66 g de diéthylamino-2 éthanethiol, on obtient après purification par chromatographie « flash » [éluant : chloroforme-méthanol (95-5 en volumes)] et concentration à sec des fractions 9 à 18 sous pression réduite (2,7 kPa) à 30 °C, 0,8 g de (diéthylamino-2 éthyl) thiométhyl-5δ pristinamycine $I_A$ sous forme d'une poudre beige fondant à 230 °C.

Spectre RMN :

0,65 (dd, 1H : 5$β_2$)

2,38 (d, 1H : 5$β_1$)

2,3 à 2,8 (m, 8H : -S $C\underline{H}_2C\underline{H}_2$ 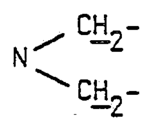 )

3,15 (dd, 1H : -$C\underline{H}_2$S-)

3,35 (dd, 1H : -$C\underline{H}_2$S-)

5,01 (dd, 1H : 5$ε_1$)

7,81 (dd, 1H x 0,9 : 1'$H_6$ 1er isomère)

7,90 (dd, 1H x 0,1 : 1'$H_6$ 2ème isomère)

On obtient une solution aqueuse à 5 % de (diéthylamino-2 éthyl) thiométhyl-5δ pristinamycine $I_A$ (produit $AF_1$) à l'état de chlorhydrate, avec :

| | |
|---|---|
| produit $AF_1$ | 30 mg |
| acide chlorhydrique 0,1N | 0,29 $cm^3$ |
| eau distillée | qsp 0,6 $cm^3$ |

Exemple 8

En opérant d'une manière analogue à celle décrite à l'exemple 1 mais à partir de 4,36 g de méthylène-5δ pristinamycine $I_A$ et de 2,2 g de diéthylamino-1 propanethiol-2, on obtient après purification par chromatographie « flash » [éluant : chloroforme-méthanol (96-4 en volumes)] et concentration à sec des fractions 10 à 25 sous pression réduite (2,7 kPa) à 30 °C, 1 g de (diéthylamino-1 propyl-2) thiométhyl-5δ pristinamycine $I_A$ sous forme d'une poudre beige fondant vers 190 °C.

Spectre RMN :

0,64 (dd, 1H : 5$β_2$)

1 à 1,2 (m, $C\underline{H}_3$-CH< et -$CH_2$-$C\underline{H}_3$)

2,37 (d, 1H : $5\beta_1$)

2,3 à 2,7 (m, 6H : 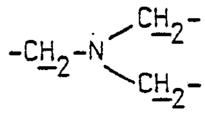 )

3,15 (dd, 1H : $-C\underline{H}_2-S$)

3,35 (dd, 1H : $-C\underline{H}_2-S$)

5,02 (dd, 1H : $5\varepsilon_1$)

7,85 (m, 1H x 0,9 : l'$H_6$ 1er isomère)

7,93 (m, 1H x 0,1 : l'$H_6$ 2ème isomère)

On obtient une solution à 4 % de (diéthylamino-1 propyl-2) thiométhyl-5δ pristinamycine $I_A$ (produit $AF_2$) à l'état de chlorhydrate, avec :

| | |
|---|---|
| produit $AF_2$ | 30 mg |
| acide chlorhydrique 0,1N | 0,29 $cm^3$ |
| eau distillée | qsp 0,75 $cm^3$ |

Le diéthylamino-1 éthanethiol-2 peut être préparé selon la méthode décrite par R. T. Wragg, J. Chem. Soc. (C), 2087 (1969).

## Exemple 9

En opérant d'une manière analogue à celle décrite à l'exemple 1, mais à partir de 3,2 g de méthylène-5δ pristinamycine $I_A$ et de 2,8 g de N,N bis(mercapto-2 éthyl) N méthylamine, on obtient après purification par chromatographie « flash » [éluant : chlorure de méthylène-méthanol (98-2 en volumes)] et concentration à sec des fractions 7 à 20 sous pression réduite (2,7 kPa) à 30 °C, 1 g de {[N(mercapto-2 éthyl) N méthylamino]-2 éthyl} thiométhyl-5δ pristinamycine $I_A$ sous forme d'une poudre blanche fondant vers 100 °C.

Spectre RMN :

0,61 (dd, 1H : $5\beta_2$)

2,29 (s, 3H : $>N-C\underline{H}_3$)

2,38 (d, 1H : $5\beta_1$)

2,3 à 2,7 (m, 8H : $-S\ C\underline{H}_2C\underline{H}_2\ N-C\underline{H}_2C\underline{H}_2\ SH$)

3,15 (dd, 1H : $-C\underline{H}_2S-$)

3,35 (dd, 1H : $-C\underline{H}_2S-$)

5,03 (dd, 1H : $5\varepsilon_1$)

7,83 (dd, 1H x 0,9 : l'$H_6$ 1er isomère)

7,93 (dd, 1H x 0,1 : l'$H_6$ 2ème isomère)

On obtient une solution aqueuse à 1 % de {[N-(mercapto-2 éthyl) N-méthylamino]-2 éthyl} thiométhyl-5δ pristinamycine $I_A$ (produit $AF_3$) à l'état de chlorhydrate, avec :

| | |
|---|---|
| produit $AF_3$ | 20 mg |
| acide chlorhydrique 0,1N | 0,38 $cm^3$ |
| eau distillée | qsp 2 $cm^3$ |

## Exemple 10

En opérant d'une manière analogue à celle décrite à l'exemple 1, mais à partir de 7,7 g de méthylène-5δ pristinamycine $I_A$ et de 9 g de bis(mercapto-2 éthyl)-1,4 pipérazine, on obtient après purification par chromatographie « flash » [éluant : chlorure de méthylène-méthanol (95-5 en volumes)] et concentration à sec des fractions 12 à 40 sous pression réduite (2,7 kPa) à 30 °C, 4,2 g de {[(mercapto-2 éthyl)-4 pipérazinyl]-2 éthyl} thiométhyl-5δ pristinamycine $I_A$ sous forme d'une poudre jaune fondant vers 165 °C.

Spectre RMN :

0,61 (dd, 1H : $5\beta_2$)
2,37 (dd, 1H : $5\beta_1$)
2,30 à 2,80 (m, 18H : $-S-\underline{CH_2}\underline{CH_2}-$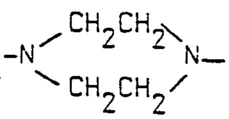$-CH_2CH_2$ SH )
3,17 (dd, 1H : $-\underline{CH_2}-S-$)
3,35 (dd, 1H : $-\underline{CH_2}-S-$)
5,03 (dd, 1H : $5\varepsilon_1$)
7,85 (m, 1H x 0,85 : 1'$H_6$ 1er isomère)
7,95 (m, 1H x 0,15 : 1'$H_6$ 2ème isomère)

On obtient une solution aqueuse à 2 % de {[(mercapto-2 éthyl)-4 pipérazinyl]-2 éthyl} thiométhyl-5δ pristinamycine $I_A$ (produit $AF_4$), à l'état de chlorhydrate, avec :

| | |
|---|---|
| produit $AF_4$ | 20 mg |
| acide chlorhydrique 0,1N | 0,36 $cm^3$ |
| eau distillée | qsp 1 $cm^3$ |

La bis(mercapto-2 éthyl)-1,4 pipérazine peut être préparée selon la méthode décrite par D. D. Reynolds, M. K. Massad, D. L. Fields et D. L. Johnson, J. Org. Chem. 26, 5111 (1961).

## Exemple 11

A une solution de 5,5 g de diméthylaminométhylène-5δ pristinamycine $I_A$ dans 60 $cm^3$ d'acide acétique on ajoute goutte à goutte 5,3 g de diméthylamino-2 éthylamine de manière à ne pas dépasser 25 °C. La solution obtenue est agitée pendant 20 heures à une température voisine de 20 °C, puis est versée lentement dans une solution aqueuse saturée de bicarbonate de sodium ; le mélange obtenu est extrait 2 fois par 750 $cm^3$ au total de chlorure de méthylène. Les phases organiques sont réunies, séchées sur du sulfate de magnésium, filtrées et concentrées à sec sous pression réduite (2,7 kPa) à 30 °C. Le résidu est purifié par chromatographie « flash » [éluant : chloroforme-méthanol (90-10 en volume)] ; les fractions 10 à 12 sont concentrées à sec sous pression réduite (2,7 kPa) à 30 °C. On obtient ainsi 3 g de (diméthylamino-2 éthyl) aminométhylène-5δ pristinamycine $I_A$ sous forme d'une poudre beige fondant vers 180 °C.

Spectre RMN :

0,90 (mt, 4H : 2γ + $5\beta_2$)
2,25 (mt, 6H : $-N(CH_3)_2$)
2,50 (mt, 3H : $-CH_2N\langle$ + $5\beta_1$)
3,25 (mt, 2H : $-N-\underline{CH_2}-$)
3,50 (mt, 2H : $5\varepsilon_2$ + $3\delta_1$)
4,90 (mt, 1 : $5\varepsilon_1$)
entre 7,15 et 7,4 (m, 1H : $=C\langle^{NH-}_{\underline{H}}$ )

9,90 (mt, 1H (échangeable $D_2O$): $-N\underline{H}-$)

On obtient une solution aqueuse à 1 % de (diméthylamino-2 éthyl) aminométhylène-5δ pristinamycine $I_A$ (produit AG), avec :

| | |
|---|---|
| produit AG | 0,1 g |
| eau distillée | qsp 10 $cm^3$ |

Exemple 12

En opérant d'une manière analogue à celle décrite à l'exemple 11, mais à partir de 1,84 g de diméthylaminométhylène-5δ pristinamycine $I_A$ et 2,8 $cm^3$ de diéthylamino-2 éthylamine et après purification par chromatographie « flash » [éluant : chloroforme-méthanol (96-4 en volumes)] et concentration à sec des fractions 9 à 13 sous pression réduite (2,7 kPa) à 30 °C, on obtient 1 g de (diéthylamino-2 éthyl) aminométhylène-5δ pristinamycine $I_A$ sous forme d'une poudre jaune fondant vers 150 °C.

Spectre RMN :

0,9 (mt, 4H : 2γ + $5\beta_2$)
1,1 (mt, 6H : $-N(CH_2-\underline{CH_3})_2$)
2,45 (d, 1H : $5\beta_1$)
3,1 à 3,4 (m, 6H : 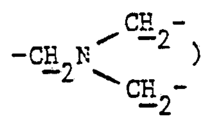 )
3,50 (mt, 2H : $5\varepsilon_2$ + $3\delta_1$)
4,90 (m, 1H : $5\varepsilon_1$)
9,9 (m, 1H (échangeable) : =CH-N$\underline{H}$-)

On obtient une solution aqueuse à 5 % de (diéthylamino-2 éthyl) aminométhylène-5δ pristinamycine $I_A$ (produit AH), à l'état de chlorhydrate, avec :

| | |
|---|---|
| produit AH | 0,1 g |
| acide chlorhydrique 0,1N | 1 $cm^3$ |
| eau distillée | qsp 2 $cm^3$ |

Exemple 13

En opérant d'une manière analogue à celle décrite à l'exemple 11, mais à partir de 2,76 g de diméthylaminométhylène-5δ pristinamycine $I_A$ et 2,22 g de N-méthyléthylènediamine et après purification par chromatographie « flash » [éluant : chloroforme-méthanol (95-5 en volumes)] et concentration à sec des fractions 16 à 20 sous pression réduite (2,7 kPa) à 30 °C, on obtient 1,3 g de (méthylamino-2 éthyl aminométhylène-5δ pristinamycine $I_A$ sous forme d'une poudre jaune fondant à 174 °C.

Spectre RMN :

0,90 (m, 4H : $5\beta_2$)
2,50 (m, 1H : $5\beta_1$)
2,7-3,6 (m, 4H : $-NH-(\underline{CH_2})_2NH-$)
3,0 (sous un massif, s, 3H : $-NH\underline{CH_3}$)
7,15 -7,40 (m, 1H : =C$\underline{H}$NH-)
9,90 (m, 1H : =CH-N$\underline{H}$CH$_3$)

On obtient une solution aqueuse à 1 % de (méthylamino-2 éthyl) aminométhylène-5δ pristinamycine $I_A$ (produit AI), à l'état de chlorhydrate, avec :

| | |
|---|---|
| produit AI | 0,03 g |
| acide chlorhydrique 0,1N | 0,31 $cm^3$ |
| eau distillée | qsp 3 $cm^3$ |

Exemple 14

En opérant d'une manière analogue à celle décrite à l'exemple 11, mais à partir de 1,84 g de diméthylaminométhylène-5δ pristinamycine $I_A$ et 2,5 $cm^3$ de diméthylamino-3 propylamine et après

purification par chromatographie « flash » [éluant : chloroforme-méthanol (90-10 en volumes)] et concentration à sec des fractions 12 à 15 sous pression réduite (2,7 kPa) à 155 °C, on obtient 0,7 g de (diméthylamino-3 propyl) aminométhylène-5δ pristinamycine $I_A$ sous forme d'une poudre jaune fondant vers 155 °C.

Spectre RMN :

0,80 à 1,05 (mt, 4H : 2γ + 5$β_2$)
1,80 (mt, 2H : $-CH_2C\underline{H}_2-CH_2-$)
2,35 (s, 6H x 0,85 : $-N(CH_3)_2$ 1er isomère)
2,40 (s, 6H x 0,15 : $-N(CH_3)_2$ 2ème isomère)
2,40 à 2,60 (mt, 3H : 5$β_1$ + $-CH_2-N<$)
3,30 (mt, 2H : $-NH-C\underline{H}_2-$)
3,50 (mt, 2H : 3$δ_1$ + 5$ε_2$)
4,90 (mt, 1H : 5$ε_1$)
9,65 (m, 1H x 0,15 : $=CH-N\underline{H}-$2ème isomère)
9,90 (m, 1H x 0,85 : $=CH-N\underline{H}-$1er isomère)

On obtient une solution à 6,6 % de (diméthylamino-3 propyl) aminométhylène-5δ pristinamycine $I_A$ (produit AJ), à l'état de chlorhydrate, avec :

| | |
|---|---|
| produit AJ | 0,1 g |
| acide chlorhydrique 0,2N | 0,51 $cm^3$ |
| eau distillée | qsp 1,5 $cm^3$ |

## Exemple 15

En opérant d'une manière analogue à celle décrite à l'exemple 11, mais à partir de 2,76 g de diméthylaminométhylène-5δ pristinamycine $I_A$ et 3,06 g de diméthylamino-1 propylamine-2 et après purification par chromatographie « flash » [éluant : chloroforme-méthanol (99-1 en volumes)] et concentration à sec des fractions 11 à 22 sous pression réduite (2,7 kPa) à 30 °C, on obtient 1,0 g de (diméthylamino-3 propyl-2) aminométhylène-5δ pristinamycine $I_A$ sous forme d'une poudre jaune fondant vers 160 °C.

Spectre RMN :

1,05 (d, 3H : $-CH-C\underline{H}_3$)
2,30 (s, 6H : $-CH_2-N(C\underline{H}_3)_2$)
2,45 (d, 1H : 5$β_1$)
2,80 (m, 1H : $-C\underline{H}CH_3$)
3,30 (sous un massif : $-NH-C\underline{H}_2-$)
3,45 (m, 2H : 5$ε_2$ + 3$δ_1$)
4,90 (m, 1H : 5$ε_1$)
7,15-7,40 (m, 1H : $=C\underline{H}NH-$)
9,90 (m, 1H : $=CH-N\underline{H}-$)

On obtient une solution aqueuse à 1 % de (diméthylamino-3 propyl-2) aminométhylène-5δ pristinamycine $I_A$ (produit AK), à l'état de chlorhydrate, avec :

| | |
|---|---|
| produit AK | 20 mg |
| acide chlorhydrique 0,1N | 0,2 $cm^3$ |
| eau distillée | qsp 2 $cm^3$ |

Exemple 16

En opérant d'une manière analogue à celle décrite à l'exemple 11, mais à partir de 2,76 g de diméthylaminométhylène-5δ pristinamycine $I_A$ et 1,53 g de diméthylamino-2 propylamine et après purification par chromatographie « flash » [éluant : chloroforme-méthanol (95-5 en volumes)] et concentration à sec des fractions 10 à 14 sous pression réduite (2,7 kPa) à 30 °C, on obtient 0,85 g de (diméthylamino-2 propyl) aminométhylène-5δ pristinamycine $I_A$ sous forme d'une poudre orange fondant vers 175 °C.

Spectre RMN :

0,90 (m, 4H : 2γ + 5$\beta_2$)

1,05 (d, 3H : >CH-$C\underline{H}_3$)

2,30 (s, 6H : -CH($CH_3$)N($C\underline{H}_3$)$_2$)

2,45 (d, 1H : 5$\beta_1$)

2,80 (m, 1H : -$\underline{C}H$C$H_3$)

3,30 (sous un massif, 2H : -NH-$C\underline{H}_2$-)

3,45 (m, 2H : 5$\varepsilon_2$ + 3$\delta_1$)

4,90 (m, 1H : 5$\varepsilon_1$)

7,15-7,40 (m, 1H : =C$\underline{H}$NH-)

9,90 (m, 1H : =CH-N$\underline{H}$-)

On obtient une solution aqueuse à 10 % (diméthylamino-2 propyl) aminométhylène-5δ pristinamycine $I_A$ (produit AL), à l'état de chlorhydrate, avec :

| | |
|---|---|
| produit AL | 0,03 g |
| acide chlorhydrique 0,1N | 0,31 $cm^3$ |

Exemple 17

En opérant d'une manière analogue à celle décrite à l'exemple 11, mais à partir de 1,84 g de diméthylaminométhylène-5δ pristinamycine $I_A$ et 3,16 g d'amino-2 diéthylamino-5 pentane et après purification par chromatographie « flash » [éluant : chloroforme-méthanol (90-10 en volumes)] et concentration à sec des fractions 15 à 27 sous pression réduite (2,7 kPa) à 30 °C, on obtient 0,9 g de (diéthylamino-5 pentyl-2) aminométhylène-5δ pristinamycine $I_A$ sous forme d'une poudre beige fondant vers 160 °C.

Spectre RMN :

1,00 (dd, 1H : 5$\beta_2$)

1,25 (mt, 6H : -N($CH_2C\underline{H}_3$)$_2$)

2,45 (d, 1H : 5$\beta_1$)

2,7-3,0 (m, 6H : -$C\underline{H}_2$-N< $C\underline{H}_2$- / $C\underline{H}_2$- )

3,45 (dd, 1H : 5$\varepsilon_2$)

7,30 (sous les aromatiques : =C$\underline{H}$-NH-)

7,85 (dd, 1H : 1'$H_6$

10 (m large, 1H : -N$\underline{H}$-CH-)

On obtient une solution aqueuse de 1 % de (diéthylamino-5 pentyl-2) aminométhylène-5δ pristinamycine $I_A$ (produit AM), à l'état de chlorhydrate, avec :

| | |
|---|---|
| produit AM | 0,02 g |
| acide chlorhydrique 0,1N | 0,2 $cm^3$ |
| eau distillée | qsp 2 $cm^3$ |

Exemple 18

En opérant d'une manière analogue à celle décrite à l'exemple 11, mais à partir de 1,84 g de diméthylaminométhylène-5δ pristinamycine $I_A$ et 2,28 g de N-(amino-2 éthyl) pyrrolidine et après purification par chromatographie « flash » [éluant : chloroforme-méthanol (98-2 en volumes)] et concentration à sec des fractions 15 à 24 sous pression réduite (2,7 kPa) à 30 °C, on obtient 0,95 g de (pyrrolidino-2 éthyl) aminométhylène-5δ pristinamycine $I_A$ sous forme d'une poudre jaune fondant à 183 °C.

Spectre RMN :

0,90 (mt, 4H : 2γ + $5\beta_2$)

1,80 (mt, 4H : 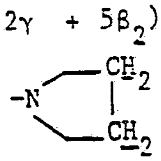 )

2,70 (mt, 6H : $-\underline{CH_2}-$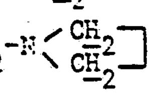 )

3,45 (m, 4H : $-NH-\underline{CH_2}-$ + $5\varepsilon_2$ + $3\delta_1$)

4,90 (m, 1H : $5\varepsilon_1$)

7,2-7,4 (m : Ar + $1'H_4$ + $1'H_5$ + $=\underline{CH}-$)

9,90 (mt, 1H : $=CHN\underline{H}CH_2-$)

On obtient une solution à 1 % de (pyrrolidino-2 éthyl) aminométhylène-5δ pristinamycine $I_A$ (produit AN), à l'état de chlorhydrate, avec :

| | |
|---|---|
| produit AN | 0,02 g |
| acide chlorhydrique 0,1N | 0,2 $cm^3$ |
| eau distillée | qsp 2 $cm^3$ |

Exemple 19

En opérant d'une manière analogue à celle décrite à l'exemple 11, mais à partir de 2,76 g de diméthylaminométhylène-5δ pristinamycine $I_A$ et 1,92 g de N-(amino-3 propyl) pyrrolidine et après purification par chromatograhie « flash » [éluant : chloroforme-méthanol (95-5 en volumes)] et concentration à sec des fractions 10 à 15 sous pression réduite (2,7 kPa) à 30 °C, on obtient 1,25 g de (pyrrolidino-3 propyl) aminométhylène-5δ pristinamycine $I_A$ sous forme d'une poudre jaune fondant à 170 °C.

Spectre RMN :

0,95 (m, 1H : $5\beta_2$)

1,95 (m, 7H : 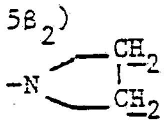 + $3\beta_1$ + $-CH_2-\underline{CH_2}CH_2N<$ )

2,45 (d large, 1H : $5\beta_1$)

2,80 (sous un massif, 6H : $-\underline{CH_2}-$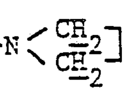 )

3,30 (mt, 2H : $-NH-\underline{CH_2}-$)

3,50 (mt, 2H : $3\delta_1$ + $5\varepsilon_2$)

4,90 (m, 1H : $5\varepsilon_1$)

7,15-7,40 (m, 1H : $=\underline{CH}NH-$)

9,90 (mt, 1H : $=CH-N\underline{H}-$)

On obtient une solution aqueuse à 1 % de (pyrrolidino-3 propyl) aminométhylène-5δ pristinamycine $I_A$ (produit AO), avec :

| | |
|---|---|
| produit AO | 0,03 g |
| eau distillée | 3 cm³ |

## Exemple 20

En opérant d'une manière analogue à celle décrite à l'exemple 11, mais à partir de 2,76 g de diméthylamino-5δ pristinamycine $I_A$ et 3,85 g de N-(amino-2 éthyl) pipéridine et après purification par chromatographie « flash » [éluant : chloroforme-méthanol (99-1 en volumes)] et concentration à sec des fractions 13 à 17 sous pression réduite 2,7 kPa) à 30 °C, on obtient 1,5 g de (pipéridino-2 éthyl) aminométhylène-5δ pristinamycine $I_A$ sous forme d'une poudre jaune fondant vers 162 °C.

Spectre RMN :

0,90 (m, 4H : 2γ + $5\beta_2$)

1,60 (mt, 6H : 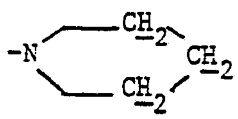 )

2,40 (m, 6H : $-H_2C-$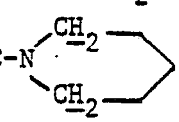 )

2,7-3,5 (m sous un massif, 2H : $-NH-CH_2-$)

3,45 (mt, 2H : $3\delta_1$ + $5\varepsilon_2$)

4,90 (mt, 1H : $5\varepsilon_1$)

7,15-7,40 (m, 1H : =CHNH-)

9,90 (mt, 1H : =CH-NH-)

On obtient une solution aqueuse à 1 % de (pipéridino-2 éthyl) aminométhylène-5δ pristinamycine $I_A$ (produit AP), à l'état de chlorhydrate, avec :

| | |
|---|---|
| produit AP | 0,02 g |
| acide chlorhydrique 0,1N | 0,2 cm³ |
| eau distillée | qsp 2 cm³ |

## Exemple 21

En opérant d'une manière analogue à celle décrite à l'exemple 11, mais à partir de 2,6 g de N-(amino-2 éthyl) morpholine et 1,84 g de diméthylaminométhylène-5δ pristinamycine $I_A$ et après purification par chromatographie « flash » [éluant : chloroforme-méthanol (95-5 en volumes)] et concentration à sec des fractions 21 à 30 sous pression réduite (2,7 kPa) à 30 °C, on obtient 0,8 g de (morpholino-2 éthyl) aminométhylène-5δ pristinamycine $I_A$ sous forme d'une poudre beige fondant vers 172 °C.

Spectre RMN :

0,95 (m, 1H : $5\beta_2$)

2,50 (m, 7H : $5\beta_1$ + $-CH_2$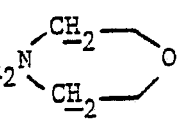 )

3,30 (m, 2H : $-NH-CH_2-$)

3,50 (m, 2H : $5\varepsilon_2$ + $3\delta_1$)

3,70 (mt, 4H : 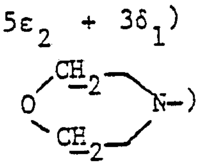 )

4,90 (m, 1H : $5\varepsilon_1$)

7,2-7,4 (m, 1H : $=C\underline{H}-$)

9,90 (mt, 1H : $=CH-N\underline{H}-CH_2-$ )

On obtient une solution aqueuse à 1 % de (morpholino-2 éthyl) aminométhylène-5δ pristinamycine $I_A$ (produit AQ), à l'état de chlorhydrate, avec :

| | |
|---|---|
| produit AQ | 0,02 g |
| acide chlorhydrique 0,1N | 0,2 $cm^3$ |
| eau distillée | qsp 2 $cm^3$ |

Exemple 22

En opérant d'une manière analogue à celle décrite à l'exemple 11, mais à partir de 2,76 g de diméthylaminométhylène-5δ pristinamycine $I_A$ et 3,66 g d'aminométhyl-2 éthyl-1 pyrrolidine et après purification par chromatographie « flash » [éluant : chloroforme-méthanol (99-1 en volumes)] et concentration à sec des fractions 10 à 14 sous pression réduite (2,7 kPa) à 30 °C, on obtient 1,3 g d'(éthyl-1 pyrrolidinyl-2) méthylaminométhylène-5δ pristinamycine $I_A$ sous forme d'une poudre beige fondant vers 160 °C.

Spectre RMN :

1,10 (t, 3H : $-CH_2-C\underline{H}_3$)

1,60 (m, 4H : 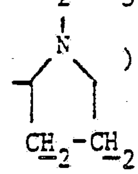 )

1,95 (m, 1H : $=CH-N\underline{H}-$)

2,8-3,6 (m, 4H : $-C\underline{H}_2N<$ + $-C\underline{H}_2NH-$)

7,15-7,40 (m, 1H : $=C\underline{H}NH-$)

On obtient une solution aqueuse à 1 % d'(éthyl-1 pyrrolidinyl-2) méthylaminométhylène-5δ pristinamycine $I_A$ (produit AR), à l'état de chlorhydrate, avec :

| | |
|---|---|
| produit AR | 0,02 g |
| acide chlorhydrique 0,1N | 0,2 $cm^3$ |
| eau distillée | qsp 2 $cm^3$ |

Exemple 23

En opérant d'une manière analogue à celle décrite à l'exemple 11, mais à partir de 2,77 g de diméthylaminométhylène-5δ pristinamycine $I_A$ et 3,4 g d'amino-3 méthyl-1 pipéridine et après purification par chromatographie « flash » [éluant : chloroforme-méthanol (95-5 en volumes)] et concentration à sec des fractions 7 à 16 sous pression réduite (2,7 kPa) à 30 °C, on obtient 0,8 g de (méthyl-1 pipéridyl-3) aminométhylène-5δ pristinamycine $I_A$ sous forme d'une poudre beige fondant à 177 °C.

Spectre RMN :

0,90 (mt, 4H : $2\gamma$ + $5\beta_2$)

1,5-2,10 (mt, 7H : $2\beta_1$ + $2\beta_2$ + $3\gamma_1$ + 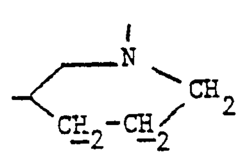 )

2,30 (s, 3H : 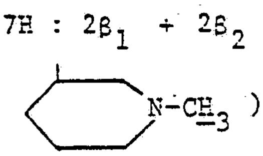 )

2,45 (d, 1H : $5\beta_1$)

2,65 (mt, 1H : -CH 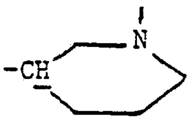 )

2,90 (mt, 4H : $4\beta_2$ + 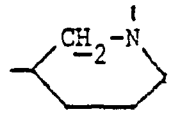 ou 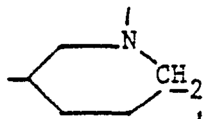 )

3,20 (mt, 7H : $-NCH_3$ en 4 + $3\delta_2$ + $4\beta_1$ + 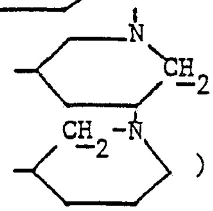 )

7,15-7,40 (m, 1H : =CHNH-)

7,80 (mt, 1H : $1'H_6$)

9,90 (mt, 1H : =CH-NH- )

11,60 (s large, 1H : OH)

On obtient une solution aqueuse à 1 % de (méthyl-1 pipéridyl-3) aminométhylène-5δ pristinamycine $I_A$ (produit AS), à l'état de chlorhydrate, avec :

| | |
|---|---|
| produit AS | 0,02 g |
| acide chlorhydrique 0,1N | 0,2 cm³ |
| eau distillée | qsp 2 cm³ |

L'amino-3 méthyl-1 pipéridine peut être préparée selon la méthode décrite par L. M. Werbel, A. Curry, E. F. Elslager, C. Hess, J. Heterocyclic Chem. *10,* 363 (1973).

## Exemple 24

En opérant d'une manière analogue à celle décrite à l'exemple 11, mais à partir de 13,8 g de diméthylaminométhylène-5δ pristinamycine $I_A$ et 3,4 g d'amino-4 méthyl-1 pipéridine et après purification par chromatographie « flash » [éluant : chloroforme-méthanol (92,5-7,5 en volumes)] et concentration à sec des fractions 15 à 20 sous pression réduite (2,7 kPa) à 30 °C, on obtient 4,0 g de (méthyl-1 pipéridyl-4) aminométhylène-5δ pristinamycine $I_A$ sous forme d'une poudre jaune fondant à 208 °C.

Spectre RMN :

0,40 (m, 4H : $2\gamma + 2\beta_2$)

2,0 (m, 4H : 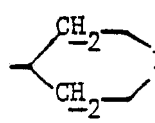 )

2,35 (s, 3H : $>N-CH_3$)

2,45 (d, 1H : $5\beta_1$)

2,90 ( 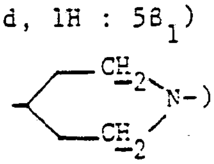 )

3,20 (sous un massif, 1H : -CH 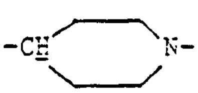 )

3,50 (d, 1H : $5\varepsilon_2$)

4,85 (sous un massif, 1H : $5\varepsilon_1$)

6,65 (d, 1H : =C$\underline{H}$NH-)

9,70 (dd, 1H x 0,25 : =CH-N$\underline{H}$- 1er isomère)

10,03 (dd, 1H x 0,85 : =CH-N$\underline{H}$- 2ème isomère)

On obtient une solution aqueuse à 10 % de (méthyl-1 pipéridyl-4) aminométhylène-5δ pristinamycine $I_A$ (produit AT), à l'état de chlorhydrate, avec :

| | |
|---|---|
| produit AT | 0,03 g |
| acide chlorhydrique 0,1N | 0,3 cm³ |
| eau distillée | qsp 0,3 cm³ |

L'amino-4 méthyl-1 pipéridine peut être préparée par la méthode décrite par E. F. Elslager, L. M. Werbel, A. Curry, N. Headen, J. Johnson, J. Med. Chem. *17*, 99 (1974).

Exemple 25

En opérant d'une manière analogue à celle décrite à l'exemple 11, mais à partir de 0,8 g de diméthylaminométhylène-5δ virginiamycine S et 1,02 g d'amino-4 méthyl-1 pipéridine et après purification par chromatographie « flash » [éluant ; chloroforme-méthanol (90-10 en volumes)] et concentration à sec des fractions 3 à 7 sous pression réduite (2,7 kPa) à 30 °C, on obtient 0,3 g de (méthyl-1 pipéridyl-4) aminométhylène-5δ virginiamycine S sous forme d'une poudre beige fondant vers 195 °C.

Spectre RMN :

0,9 (m, 4H : 2γ + 5$\beta_2$)

2,30 (s, 3H : 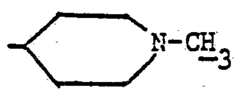 )

2,80 à 3,30 (m, 5H :-HN 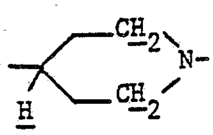 )

3,55 (dd, 1H : 5$\varepsilon_2$)

4,90 (m, 1H : 5$\varepsilon_1$)

7,10 à 7,40 (m : aromatiques + =C$\underline{H}$-NH-)

7,70 (dd, 1H : 1'$H_6$)

10,1 (m, 1H : =CH-N$\underline{H}$-)

On obtient une solution aqueuse à 5 % de (méthyl-1 pipéridyl-4) aminométhylène-5δ virginiamycine S (produit AU), à l'état de chlorhydrate, avec :

| | |
|---|---|
| produit AU | 0,1 g |
| acide chlorhydrique 0,1N | 1,05 cm³ |
| eau distillée | qsp 2 cm³ |

Exemple 26

En opérant d'une manière analogue à celle décrite à l'exemple 11, mais à partir de 2,76 g de diméthylaminométhylène-5δ pristinamycine $I_A$ et 2,15 g d'(amino-2 éthyl)-1 méthyl-4 pipérazine et après purification par chromatographie « flash » [éluant : chloroforme-méthanol (95-5 en volumes)] et concentration à sec des fractions 10 à 16 sous pression réduite (2,7 kPa) à 30 °C, on obtient 0,9 g de [(méthyl-4 pipérazinyl-1)-2 éthyl] aminométhylène-5δ pristinamycine $I_A$ sous forme d'une poudre jaune fondant à 150 °C.

Spectre RMN :

1,00 (m, 1H : 5$\beta_2$)

2,30 (s, 3H : >N-$CH_3$)

2,50 (m, 9H : $-CH_2-$ pipérazine + $5\beta_1$)

2,90 (sous un massif : $-CH_2-\underline{CH}_2-N<$)

3,30 (m, 2H : $-NH-\underline{CH}_2-$)

3,50 (m, 2H : $5\varepsilon_2$ + $3\delta_1$)

4,90 (m, 1H : $5\varepsilon_1$)

7,15-7,40 (m, 1H : $=\underline{CH}NH-$)

9,90 (m, 1H : $=CH-\underline{NH}-$)

On obtient une solution aqueuse à 10 % de [(méthyl-4 pipérazinyl-1)-2 éthyl] aminométhylène-5δ pristinamycine $I_A$ (produit AV), à l'état de chlorhydrate, avec :

| | |
|---|---|
| produit AV | 15 mg |
| acide chlorhydrique 0,1N | 0,15 $cm^3$ |

L'(amino-2 éthyl)-1 méthyl-4 pipérazine peut être préparée de la manière suivante :

A une solution de 10,0 g de bromhydrate de bromo-2 éthylamine dans 60 $cm^3$ d'éthanol absolu, on ajoute 9,75 g de N-méthyl-pipérazine. La solution obtenue est agitée pendant 16 heures à une température voisine de 20 °C puis l'éthanol est éliminé sous pression réduite (2,7 kPa) à 30 °C. Le résidu huileux est repris par 50 $cm^3$ de chloroforme ; le mélange obtenu est agité avec 20 $cm^3$ d'une solution aqueuse de soude 10N. La phase aqueuse est extraite 3 fois avec 150 $cm^3$ au total de chloroforme. Les phases organiques sont réunies, séchées sur du sulfate de sodium, filtrées et concentrées à sec sous pression réduite (2,7 kPa) à 30 °C. Le résidu est distillé sous pression réduite (2,7 kPa) ; on obtient ainsi 4,5 g d'(amino-2 éthyl)-1 méthyl-4 pipérazine sous forme d'une huile jaune [PE (2,7 kPa) = 118-119 °C].

## Exemple 27

En opérant d'une manière analogue à celle décrite à l'exemple 11, mais à partir de 4,0 g de diméthylaminométhylène-5δ pristinamycine $I_A$ et de 0,55 g d'histamine et après purification par chromatographie « flash » [éluant : chloroforme-méthanol (95-5 en volumes)] et concentration à sec des fractions 25 à 50 sous pression réduite (2,7 kPa) à 30 °C, on obtient 2,04 g d'[(imidazolyl-4)-2 éthyl] aminométhylène-5δ pristinamycine $I_a$ sous forme d'une poudre jaune fondant à 138 °C.

Spectre RMN :

0,90 (m, 4H : $2\gamma$ + $5\beta_2$)

2,40 (d large, 1H : $5\beta_1$)

2,90 (sous un massif, m, 1H : $5\varepsilon_1$)

3,50 (d, 4H : $5\varepsilon_2$ + $3\delta_1$ + $-NH-\underline{CH}_2-$)

4,80 (sous un massif, 1H : $5\varepsilon_1$)

6,65 (m, 2H : $H_5$ + $>NH$ histamine)

7,50 (s, 1H, H en 2 de l'histamine)

entre 7,15 et 7,40 (m, 1H : $=\underline{CH}NH-$)

9,65 (m, 1H x 0,15 : $=CH-\underline{NH}-$ 2ème isomère)

9,95 (m, 1H x 0,85 : $=CH-\underline{NH}-$ 1er isomère)

On obtient une solution aqueuse à 10 % d'[(imidazolyl-4)-2 éthylaminométhylène]-5δ pristinamycine $I_A$ (produit AW), à l'état de chlorhydrate avec :

| | |
|---|---|
| produit AW | 0,1 g |
| acide chlorhydrique 0,1N | qsp 1 $cm^3$ |

## Exemple 28

A une solution de 1,84 g de diméthylaminométhylène-5δ pristinamycine $I_A$ dans 40 $cm^3$ d'acide

acétique, on ajoute 2,1 g de diméthylamino-2 éthanethiol. La solution obtenue est agitée pendant 20 heures à une température voisine de 20 °C, puis est versée lentement dans une solution aqueuse saturée de bicarbonate de sodium ; le mélange obtenu est extrait 3 fois par 400 $cm^3$ au total de chlorure de méthylène. Les phases organiques sont réunies, séchées sur du sulfate de magnésium, filtrées et concentrées à sec sous pression réduite (2,7 kPa) à 30 °C. Le résidu obtenu est purifié par chromatographie « flash » [éluant : chloroforme-méthanol (96-4 en volumes)] ; les fractions 5 et 6 sont réunies et concentrées à sec sous pression réduite (2,7 kPa) à 30 °C. On obtient ainsi 0,8 g de (diméthylamino-2 éthyl) thiométhylène-5δ pristinamycine $I_A$ sous forme d'une poudre jaune fondant vers 150 °C.

Spectre RMN :

0,68 (dd, 1H : $5\beta_2$)

2,32 (s, 6H x 0,85 : $-CH_2N(C\underline{H}_3)_2$ 1er isomère)

2,35 (s, 6H x 0,15 : $-CH_2N(C\underline{H}_3)_2$ 2ème isomère)

2,45 (d, 1H : $5\beta_1$)

2,65 (mt, 2H : $-SC\underline{H}_2-$)

3,05 (t, 2H : $-C\underline{H}_2N<$)

3,43 (dd, 1H : $5\varepsilon_2$)

5,15 (dans un massif : $5\varepsilon_1$)

7,60 (s large, 1H : $=C\underline{H}S-$)

7,83 (mt, 1H : 1'$H_6$ deux isomères)

On obtient une solution aqueuse à 1 % de (diméthylamino-2 éthyl) thiométhylène-5δ pristinamycine $I_A$ (produit AX), à l'état de chlorhydrate, avec :

| | |
|---|---|
| produit AX | 0,1 g |
| acide chlorhydrique 0,1N | 1 $cm^3$ |
| eau distillée | qsp 10 $cm^3$ |

## Exemple 29

En opérant d'une manière analogue à celle décrite à l'exemple 28, mais à partir de 3,68 g de diméthylaminométhylène-5δ pristinamycine $I_A$ et 8,5 g de diéthylamino-2 éthanethiol et après purification par chromatographie « flash » [éluant : chloroforme-méthanol (96-4 en volumes)] et concentration à sec des fractions 13 à 20 sous pression réduite (2,7 kPa) à 30 °C, on obtient 0,85 g de (diéthylamino-2 éthyl) thiométhylène-5δ pristinamycine $I_A$ sous forme d'une poudre beige fondant vers 192 °C.

Spectre RMN :

0,65 (dd, 1H : $5\beta_2$)

1,05 (t, 6H : $-N(CH_2C\underline{H}_3)_2$

2,42 (d, 1H : $5\beta_1$)

2,60 (q, 4H : $-N(C\underline{H}_2CH_3)_2$)

3,42 (dd, 1H : $5\varepsilon_2$)

5,10 (sous un massif, 1H : $5\varepsilon_1$)

7,58 (s large, 1H : $=C\underline{H}-S-$)

7,82 (dd, 1H x 0,95 : 1'$H_6$ 1er isomère)

7,98 (dd, 1H x 0,05 : 1'$H_6$ 2ème isomère)

On obtient une solution aqueuse à 1 % de (diéthylamino-2 éthyl) thiométhylène-5δ pristinamycine $I_A$ (produit AY), à l'état de chlorhydrate, avec :

| | |
|---|---|
| produit AY | 0,04 g |
| acide chlorhydrique 0,1N | 0,4 $cm^3$ |
| eau distillée | qsp 4 $cm^3$ |

## Exemple 30

En opérant d'une manière analogue à celle décrite à l'exemple 28, mais à partir de 3 g de diméthylaminométhylène-5δ pristinamycine $I_A$ et 0,4 g de diméthylamino-3 propanethiol et après purification par chromatographie « flash » [éluant : chloroforme-méthanol (92,5-7,5 en volumes)] et concentration à sec des fractions 10 à 17 sous pression réduite (2,7 kPa) à 30 °C, on obtient 0,85 g de (diméthylamino-3 propyl) thiométhylène-5δ pristinamycine $I_A$ sous forme d'une poudre beige fondant vers 170 °C.

Spectre RMN :

0,70 (dd, 1H : 5$\beta_2$)
1,90 (m, 2H : $-S-CH_2\underline{CH_2}CH_2N\langle$)
2,20 (s, 6H : $-N(\underline{CH_3})_2$)
2,40 (d, 1H : 5$\beta_1$)
2,90 (m, 2H : $-\underline{CH_2}-N\langle$)
3,45 (dd, 1H : 5$\varepsilon_2$)
7,65 (s large, 1H : $=\underline{CH}-S-$)

On obtient une solution aqueuse à 1 % de (diméthylamino-3 propyl) thiométhylène-5δ pristinamycine $I_A$ (produit AZ), à l'état de chlorhydrate, avec :

| | |
|---|---|
| produit AZ | 0,03 g |
| acide chlorhydrique 0,1N | 0,3 $cm^3$ |
| eau distillée | qsp 3 $cm^3$ |

## Exemple 31

En opérant d'une manière analogue à celle décrite à l'exemple 28, mais à partir de 1,8 g de diméthylaminométhylène-5δ virginiamycine S et 0,48 g de diméthylamino-3 propanethiol et après purification par chromatographie « flash » [éluant : chloroforme-méthanol (95-5 en volumes)] et concentration à sec des fractions 5 à 14 sous pression réduite (2,7 kPa) à 30 °C, on obtient 0,7 g de (diméthylamino-3 propyl) thiométhylène-5δ virginiamycine S sous forme d'une poudre beige fondant vers 140 °C.

Spectre RMN :

0,50 (dd, 1H : 5$\beta_2$)
2 (m, 2H : $-SCH_2-\underline{CH_2}-CH_2N\langle$)
2,35 (s, 6H : $-S(CH_2)_3N(\underline{CH_3})_2$)
2,60 (t, 2H : $-S\underline{CH_2}-CH_2CH_2-N\langle$)
3 (t, 2H : $-SCH_2CH_2\underline{CH_2}N\langle$)
3,35 (dd, 1H : 5$\varepsilon_2$)
4,90 (dd, 1H : 5$\varepsilon_1$)
5,20 (m, 1H : 5α)
7,60 (s large, 1H : $=\underline{CH}-S-$)
7,80 (dd, 1H : 1'$H_6$)

On obtient une solution aqueuse à 10 % de (diméthylamino-3 propyl) thiométhylène-5δ virginiamy-

cine S (produit AAA), à l'état de chlorhydrate, avec :

| | |
|---|---|
| produit AAA | 0,1 g |
| acide chlorhydrique 0,2N | 0,52 $cm^3$ |
| eau distillée | qsp 1 $cm^3$ |

Exemple 32

En opérant d'une manière analogue à celle décrite à l'exemple 28, mais à partir de 4 g de diméthylaminométhylène-5δ pristinamycine $I_A$ et 0,7 g de diméthylamino-3 méthyl-2 propanethiol et après purification par chromatographie « flash » [éluant : chloroforme-méthanol (94-6 en volumes)] et concentration à sec sous pression réduite (2,7 kPa) à 30 °C, on obtient 0,96 g de (diméthylamino-3 méthyl-2 propyl) thiométhylène-5δ pristinamycine $I_A$ sous forme d'une poudre beige fondant à 234 °C.

Spectre RMN :

0,65 (dd, 1H : 5ε$_2$)

1,05 (d, 3H : $-\underset{|}{C}H\underline{CH_3}$)

2,25 (s, 6H : $-N(\underline{CH_3})_2$)

2,40 (d, 1H : 5β$_1$)

3,15 et 2,90 (système ABX, 2H :$-\underline{CH_2}N<$)

3,45 (d large, 2H : 5ε)

7,75 (dd, 1H x 0,80 :1'H$_6$ 1er isomère)

7,95 (dd, 1H x 0,20 :1'H$_6$ 2ème isomère)

On obtient une solution aqueuse à 1 % de (diméthylamino-3 méthyl-2 propyl) thiométhylène-5δ pristinamycine $I_A$ (produit AAB), à l'état de chlorhydrate, avec :

| | |
|---|---|
| produit AAB | 0,03 g |
| acide chlorhydrique 0,1N | 0,3 $cm^3$ |
| eau distillée | qsp 3 $cm^3$ |

Le diméthylamino-3 méthyl-2 propanethiol peut être préparé de la manière suivante :

A une solution de 5,33 g de N,N-diméthyl acétylthio-3 méthyl-2 propylamine dans 50 $cm^3$ de méthanol anhydre, on ajoute 0,026 g de sodium. Le mélange obtenu est chauffé au reflux pendant 7 heures puis le méthanol est éliminé sous pression réduite (2,7 kPa) à 50 °C. Le résidu est distillé sous pression réduite (2,7 kPa). On obtient ainsi 0,9 g de diméthylamino-3 méthyl-2 propanethiol sous forme d'une huile jaune distillant à 56 °C sous 2,7 kPa.

La N,N-diméthyl acétylthio-3 méthyl-2 propylamine peut être préparée de la manière suivante :

A une solution de 29,5 g de N,N-diméthyl chloro-1 méthyl-2 propylamine dans 120 $cm^3$ d'isopropanol, on ajoute 15,7 $cm^3$ d'acide thiolacétique. Le mélange obtenu est chauffé au reflux pendant 48 heures, puis l'isopropanol est éliminé sous pression réduite (2,7 kPa) à 60 °C. Le résidu obtenu est traité par 100 $cm^3$ d'une solution aqueuse saturée de bicarbonate de sodium et la phase aqueuse est extraite 3 fois avec 600 $cm^3$ au total d'éther éthylique. Les phases organiques sont réunies, séchées sur du sulfate de magnésium, filtrées et concentrées à sec. Le résidu obtenu est purifié par chromatographie « flash » [éluant : chloroforme-méthanol (90-10 en volumes)] ; les fractions 6 à 10 sont réunies et concentrées à sec sous pression réduite (2,7 kPa) à 30 °C. On obtient ainsi 5,57 g de N,N-diméthyl acétylthio-3 méthyl-2 propylamine sous forme d'une huile rouge.

La N,N-diméthyl chloro-1 méthyl-2 propylamine peut être préparée selon la méthode décrite par J. P. Bourquin et coll., Helv. Chim. Acta, *41*, 1072 (1958).

Exemple 33

En opérant d'une manière analogue à celle décrite à l'exemple 28, mais à partir de 4 g de diméthylaminométhylène-5δ pristinamycine $I_A$ et 1,14 g de diméthylamino-2 méthyl-2 propanethiol et après purification par chromatographie « flash » éluant : chloroforme-méthanol (92-8 en volumes)] et concentration à sec des fractions 12 à 30 sous pression réduite (2,7 kPa) à 30 °C, on obtient 1,4 g de (diméthylamino-2 méthyl-2 propyl) thiométhylène-5δ pristinamycine $I_A$ sous forme d'une poudre beige fondant vers 200 °C.

Spectre RMN :

0,55 (dd, 1H x 0,20 : 5$\beta_2$ 2ème isomère)
0,68 (dd, 1H x 0,80 : 5$\beta_2$ 1er isomère)
1,15 (s, 6H : $-C(CH_3)_2-$)
2,30 (s, 6H x 0,80: $-N(CH_3)_2$ 1er isomère)
2,42 (s, 6H x 0,20: $-N(CH_3)_2$ 2ème isomère)
2,40 (d, 1H : 5$\beta_1$)
2,80 (sous un massif : $-S-CH_2-$)
3,42 (dd, 1H : 5$\varepsilon_2$)
7,55 (s large, 1H : =CH-S)
7,80 (dd, 1H x 0,80: 1'$H_6$ 1er isomère)
7,98 (dd, 1H x 0,20: 1'$H_6$ 2ème isomère)

On obtient une solution aqueuse à 1 % de (diméthylamino-2 méthyl-2 propyl) thiométhylène-5δ pristinamycine $I_A$ (produit AAC), à l'état de chlorhydrate, avec :

| | |
|---|---|
| produit AAC | 0,03 mg |
| acide chlorhydrique | 0,3 ml |
| eau distillée | qsp 3 ml |

Le diméthylamino-2 méthyl-2 propanethiol peut être préparé selon la méthode décrite par H. R. Snyder, J. M. Stewart, J. B. Ziegler, J. Am. Chem. Soc., *69*, 2672 (1947).

Exemple 34

En opérant d'une manière analogue à celle décrite à l'exemple 28, mais à partir de 4 g de diméthylaminométhylène-5δ pristinamycine $I_A$ et 1,1 g de (pyrrolidinyl-1)-2 éthanethiol et après purification par chromatographie « flash » [éluant : chloroforme-méthanol (96-4 en volumes)] et concentration à sec des fractions 9 à 15 sous pression réduite (2,7 kPa) à 30 °C, on obtient 1,3 g de [(pyrrolidinyl-1)-2 éthyl] thiométhylène-5δ pristinamycine $I_A$ sous forme d'une poudre beige fondant vers 180 °C.

Spectre RMN :

0,65 (dd, 1H : 5$\beta_2$)
1,85 (m, 4H : 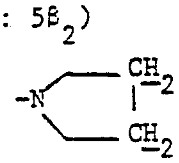 )
2,45 (d, 1H : 5$\beta_1$)
2,75 et 2,90 (m, 8H : $-CH_2-$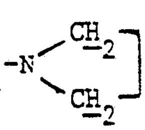 et $-SCH_2-$)
3,45 (dd, 1H : 5$\varepsilon_2$)
7,60 (s large, 1H : =CH-S-)
7,85 (dd, 1H : 1'$H_6$)

On obtient une solution aqueuse à 1 % de [(pyrrolidinyl-1)-2 éthyl] thiométhylène-5δ pristinamycine $I_A$ (produit AAD), à l'état de chlorhydrate, avec :

| | |
|---|---|
| produit AAD | 0,03 g |
| acide chlorhydrique 0,1N | 0,3 ml |
| eau distillée | qsp 3 ml |

Le (pyrrolidinyl-1)-2 éthanethiol peut être préparé selon la méthode décrite par J. W. Haeffele, R. W. Broge, Proc. Sci. Toilet Goods Assoc. *32*, 52 (1959) [Chem. Abstr. *54*, 17234e (1960)].

Exemple 35

En opérant d'une manière analogue à celle décrite à l'exemple 28, mais à partir de 4 g de diméthylaminométhylène-5δ pristinamycine $I_A$ et 1,74 g de (méthyl-1 pyrrolidinyl-2)-2 éthanethiol et après purification par chromatographie « flash » [éluant : chloroforme-méthanol (92-8 en volumes)] et concentration à sec des fractions 12 à 22 sous pression réduite (2,7 kPa) à 30 °C, on obtient 1,33 g de [(méthyl-1 pyrrolidinyl-2)-2 éthyl] thiométhylène-5δ pristinamycine $I_A$ sous forme d'une poudre beige fondant vers 215 °C.

Spectre RMN :

0,65 (dd, 1H : 5β$_2$)

1,4-2,3 (m, 6H : 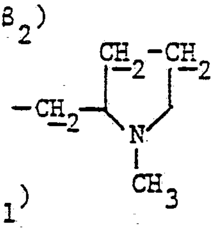 )

2,40 (d, 1H : 5β$_1$)

2,48 (s, 3H : >N-$CH_3$ pyrrolidine)

3,40 (dd, 1H : 5ε$_2$)

7,50 (s large, 1H : =CH-)

7,80 (dd, 1H x 0,85: 1'$H_6$ 1er isomère)

8,00 (dd, 1H x 0,15: 1'$H_6$ 2ème isomère)

On obtient une solution aqueuse à 0,6 % de [(méthyl-1 pyrrolidinyl-2)-2 éthyl] thiométhylène-5δ pristinamycine $I_A$ (produit AAE), à l'état de chlorhydrate, avec :

| | |
|---|---|
| produit AAE | 0,03 g |
| acide chlorhydrique 0,1N | 0,3 cm$^3$ |
| eau distillée | qsp 5 cm$^3$ |

Le méthyl-1 (pyrrolidinyl-2)-2 éthanethiol peut être préparé d'une manière analogue à celle décrite à l'exemple 32 pour préparer le diméthylamino-3 méthyl-2 propanethiol, mais à partir de 15,7 g d'(acétylthio-2 éthyl)-2 méthyl-1 pyrrolidine et 0,07 g de sodium. On obtient ainsi 12,2 g de produit sous forme d'une huile rouge.

L'(acétylthio-2 éthyl)-2 méthyl-1 pyrrolidine peut être préparée d'une manière analogue à celle décrite à l'exemple 32 pour préparer la N,N-diméthyl acétylthio-3 méthyl-2 propylamine mais à partir de 12,7 g de (chloro-2 éthyl)-2 méthyl-1 pyrrolidine et 6,8 cm$^3$ d'acide thiolacétique. On obtient ainsi 15,7 g de produit sous forme d'une huile rouge.

Exemple 36

En opérant d'une manière analogue à celle décrite à l'exemple 28, mais à partir de 3,0 g de diméthylaminométhylène-5δ pristinamycine $I_A$ et 0,48 g de mercapto-4 méthyl-1 pipéridine et après purification par chromatographie «flash» [éluant : chloroforme-méthanol (90-10 en volumes)] et concentration à sec des fractions 15 à 19 sous pression réduite (2,7 kPa) à 30 °C, on obtient 1,2 g de (méthyl-1 pipéridyl-4) thiométhylène-5δ pristinamycine $I_A$ sous forme d'une poudre jaune fondant vers 170 °C.

Spectre RMN :

0,68 (dd, 1H : 5β$_2$)

2,0-2,2 (m, 4H : 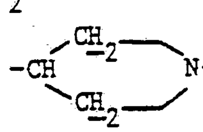 )

2,30 (s, 3H : >N-$CH_3$)

2,45 (d, 1H : $5\beta_1$)

2,85 (m, 4H : 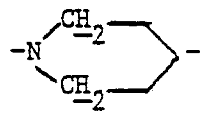 )

3,05 (mt, 1H : −S−CH− )

3,40 (dd, 1H : $5\varepsilon_2$)

5,15 (d, 1H : $5\varepsilon_1$)

7,67 (s large, 1H : =CH−S−)

7,85 (dd, 1H x 0,85 : l'$H_6$ 1er isomère)

8,0 (dd, 1H x 0,15 : l'$H_6$ 2ème isomère)

On obtient une solution aqueuse à 1 % de (méthyl-1 pipéridyl-4) thiométhylène-5δ pristinamycine $I_A$ (produit AAF), à l'état de chlorhydrate, avec :

| | |
|---|---|
| produit AAF | 0,05 g |
| acide chlorhydrique 0,1N | 0,5 $cm^3$ |
| eau distillée | qsp 5 $cm^3$ |

La mercapto-4 méthyl-1 pipéridine peut être préparée selon la méthode décrite par H. Barrer et R. E. Lyle, J. Org. Chem. *27*, 641 (1962).

Exemple 37

En opérant d'une manière analogue à celle décrite à l'exemple 28, mais à partir de 4 g de diméthylaminométhylène-5δ pristinamycine $I_A$ et 0,8 g d'éthyl-1 mercapto-3 pipéridine et après purification par chromatographie « flash » [éluant : chloroforme-méthanol (92-8 en volumes)] et concentration à sec des fractions 6 à 9 sous pression réduite (2,7 kPa) à 30 °C, on obtient 1,1 g d'(éthyl-1 pipéridyl-3) thiométhylène-5δ pristinamycine $I_A$ fondant vers 175 °C.

Spectre RMN :

0,70 (s large, 1H : $5\beta_2$)

1,20 (t, 3H : $-CH_2\underline{CH_3}$)

2,45 (d large, 1H : $5\beta_1$)

2,90 (m, 6H : 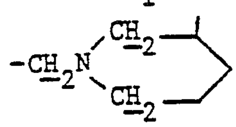 )

7,50 (s large, 1H : =CH−S−)

7,80 (dd, 1H x 0,80: l'$H_6$ 1er isomère)

7,95 (dd, 1H x 0,20: l'$H_6$ 2ème isomère)

On obtient une solution aqueuse à 1 % d'(éthyl-1 pipéridyl-3) thiométhylène-5δ pristinamycine $I_A$ (produit AAG), à l'état de chlorhydrate, avec :

| | |
|---|---|
| produit AAG | 0,03 g |
| acide chlorhydrique 0,1N | 0,3 $cm^3$ |
| eau distillée | qsp 3 $cm^3$ |

L'éthyl-1 mercapto-3 pipéridine peut être préparée selon la méthode décrite par J. H. Biel et coll., J. Am. Chem. Soc. *77*, 2250 (1955).

Exemple 38

En opérant d'une manière analogue à celle décrite à l'exemple 28, mais à partir de 3,0 g de

diméthylaminométhylène-5δ pristinamycine $I_A$ et 0,55 g de N-(mercapto-2 éthyl) N,N',N'-triméthyléthylènediamine et après purification par chromatographie « flash » [éluant : chloroforme-méthanol (90-10 en volumes)] et concentration à sec des fractions 17 à 24 sous pression réduite (2,7 kPa) à 30 °C, on obtient 1,0 g de [(diméthylamino-2 éthyl) méthylamino-2 éthyl] thiométhylène-5δ pristinamycine $I_A$ sous forme d'une poudre jaune fondant vers 160 °C.

Spectre RMN :

0,68 (dd, 1H : 5$\beta_2$)
2,30 (s, 3H : $-\overset{|}{N}C\underline{H}_3$)
2,40 (d, 1H : 5$\beta_1$)
2,4-3,1 (m, 8H : $-S(C\underline{H}_2)_2N-(C\underline{H}_2)_2N\langle$)
3,40 (dd, 1H : 5$\varepsilon_2$)
5,10 (sous un massif, 1H : 5$\varepsilon_1$)
7,58 (s large, 1H : =C$\underline{H}$-S-)
7,80 (dd, 1H : 1'$H_6$)

On obtient une solution aqueuse à 1 % de [(diméthylamino-2 éthyl) méthylamino-2 éthyl] thiométhylène-5δ pristinamycine $I_A$ (produit AAH), avec :

| | |
|---|---|
| produit AAH | 0,03 g |
| eau distillée | qsp 3 $cm^3$ |

La N-(mercapto-2 éthyl) N,N',N'-triméthyléthylènediamine peut être préparée de la manière suivante : on ajoute 5,0 g de carbonate d'éthyle et de mercapto-2 éthyle à une solution de 10,2 g de N,N',N'-triméthyléthylènediamine dans 40 $cm^3$ de toluène portée au reflux. Après 5 heures à reflux, le toluène est éliminé sous pression réduite (2,7 kPa) à 50 °C et le résidu distillé à cette pression. On obtient la N-(mercapto-2 éthyl) N,N',N'-triméthyléthylènediamine sous forme d'un liquide jaune distillant à 105 °C sous 2,7 kPa.

Le carbonate d'éthyle et de mercapto-2 éthyle peut être préparé selon la méthode décrite par D. D. Reynolds, D. L. Fields, D. L. Johnson, J. Org. Chem., *26*, 5125 (1961)

Exemple 39

En opérant d'une manière analogue à celle décrite à l'exemple 28, mais à partir de 4 g de diméthylaminométhylène-5δ pristinamycine $I_A$ et 2 g de bis(diméthylamino)-1,3 propanethiol-2 et après purification par chromatographie « flash » [éluant : chloroforme-méthanol (92-8 en volumes)] et concentration à sec des fractions 32 à 56 sous pression réduite (2,7 kPa) à 30 °C, on obtient 1,6 g de [bis(diméthylamino)-1,3 propyl-2] thiométhylène-5δ pristinamycine $I_A$ sous forme d'une poudre beige fondant vers 190 °C.

Spectre RMN :

0,55 (dd, 1H x 0,80 : 5$\beta_2$ 2ème isomère)
0,67 (dd, 1H x 0,20 : 5$\beta_2$ 1er isomère)
2,30 (m, 6H : $-N(C\underline{H}_3)_2$)
2,8-3,2 (m, 4H : $-S-CH(C\underline{H}_2N\langle)_2$)
7,62 (m, 1H : =C$\underline{H}$-S-)
7,80 (dd, 1H x 0,80 : 1'$H_6$ 1er isomère)
7,98 (dd, 1H x 0,20 : 1'$H_6$ 2ème isomère)

On obtient une solution aqueuse à 1 % de [bis(diméthylamino)-1,3 propyl-2] thiométhylène-5δ pristinamycine $I_A$ (produit AAI), à l'état de chlorhydrate, avec :

| | |
|---|---|
| produit AAI | 0,03 g |
| acide chlorhydrique 0,1N | 0,3 $cm^3$ |
| eau distillée | qsp 3 $cm^3$ |

Le bis(diméthylamino)-1,3 propanethiol-2 peut être préparé selon la méthode décrite par J. M. Stewart, J. Org. Chem., 29, 1655 (1964).

Exemple 40

En opérant d'une manière analogue à celle décrite à l'exemple 28, mais à partir de 3,0 g de diméthylaminométhylène-5δ pristinamycine $I_A$ et 0,58 g de (mercapto-2 éthyl)-1 méthyl-4 pipérazine et après purification par chromatographie « flash » [éluant : chloroforme-méthanol (87,5-12,5 en volumes)] et concentration à sec des fractions 16 à 30 sous pression réduite (2,7 kPa) à 30 °C, on obtient 1,6 g de [(méthyl-4 pipérazinyl-1)-2 éthyl] thiométhylène-5δ pristinamycine $I_A$ sous forme d'une poudre beige fondant vers 170 °C.

Spectre RMN :

0,56 (dd, 1H x 0,20: $5\beta_2$ 1er isomère)

0,68 (dd, 1H x 0,80: $5\beta_2$ 2ème isomère)

2,40 (s, 3H : $>NC\underline{H}_3$)

2,5-3 (m, 12H : $-S(C\underline{H}_2)_2N<$ + tous les $-C\underline{H}_2-$ de pipérazine)

3,42 (dd, 1H : $5\varepsilon_2$)

5,12 (d large : $5\varepsilon_1$)

7,60 (s large, 1H : $=C\underline{H}S-$)

7,80 (dd, 1H : $1'H_6$, mélange des 2 isomères)

On obtient une solution aqueuse à 1 % de [(méthyl-4 pipérazinyl-1)-2 éthyl] thiométhylène-5δ pristinamycine $I_A$ (produit AAJ), à l'état de chlorhydrate, avec :

| | |
|---|---|
| produit AAJ | 0,05 g |
| acide chlorhydrique 0,1N | 0,5 $cm^3$ |
| eau distillée | qsp 5 $cm^3$ |

La (mercapto-2 éthyl)-1 méthyl-4 pipérazine peut être préparée selon la méthode décrite par D. D. Reynolds et coll., J. Org. Chem. *26*, 5125 (1961).

Exemple 41

En opérant d'une manière analogue à celle décrite à l'exemple 28, mais à partir de 4,0 g de diméthylaminométhylène-5δ pristinamycine $I_A$ et 1,5 g de (mercapto-3 propyl)-1 méthyl-4 pipérazine et après purification par chromatographie « flash » [éluant : chloroforme-méthanol (95-5 en volumes)] et concentration à sec des fractions 24 à 41 sous pression réduite (2,7 kPa) à 30 °C, on obtient 2,06 g de [(méthyl-4 pipérazinyl-1)-3 propyl] thiométhylène-5δ pristinamycine $I_A$ sous forme d'une poudre beige fondant vers 190 °C.

Spectre RMN :

0,68 (dd, 1H : $5\beta_2$)

1,90 (mt, 2H : $-CH_2-C\underline{H}_2CH_2N<$)

2,40 (s, 3H : $>NC\underline{H}_3$)

2,3 à 2,8 (m, 8H : $-S-C\underline{H}_2-$ + $-C\underline{H}_2-$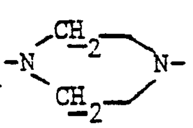 )

3,45 (m, 1H : $5\varepsilon_2$)

7,64 (s large, 1H x 0,80: =CH-S- 1er isomère)

7,70 (s large, 1H x 0,20: =CH-S- 2ème isomère)

7,80 (dd, 1H x 0,80: $1'H_6$ 1er isomère)

7,98 (dd, 1H x 0,20: $1'H_6$ 2ème isomère)

On obtient une solution aqueuse à 10 % de [(méthyl-4 pipérazinyl-1)-3 propyl] thiométhylène-5δ pristinamycine $I_A$ (produit AAK), à l'état de chlorhydrate, avec :

| | |
|---|---|
| produit AAK | 0,05 g |
| acide chlorhydrique 0,1N | qsp 0,5 ml |

La (mercapto-3 propyl)-1 méthyl-4 pipérazine peut être préparée d'une manière analogue à celle décrite dans l'exemple 32 pour préparer le diméthylamino-3 méthyl-2 propanethiol, mais à partir de 109 g d'(acétylthio-3 propyl)-1 méthyl-4 pipérazine et 0,46 g de sodium. On obtient 64,8 g de (mercapto-3 propyl)-1 méthyl-4 pipérazine sous forme d'une huile jaune distillant à 133 °C sous 0,13 kPa.

L'(acétylthio-3 propyl)-1 méthyl-4 pipérazine peut être préparée d'une manière analogue à celle décrite dans l'exemple 32 pour préparer la N,N-diméthyl acétylthio-3 méthyl-2 propylamine, mais à partir de 138 g de (chloro-3 propyl)-1 méthyl-4 pipérazine et 68,5 g d'acide thiolacétique. On obtient ainsi 109 g d'(acétylthio-3 propyl)-1 méthyl-4 pipérazine sous forme d'une huile jaune distillant vers 160 °C sous 0,13 kPa.

Exemple 42

En opérant d'une manière analogue à celle décrite à l'exemple 28, mais à partir de 4,0 g de diméthylaminométhylène-5δ pristinamycine $I_A$ et 1,3 g d'iodure de mercapto-3 méthyl-2 propylammonium et après purification par chromatographie « flash » [éluant : chloroforme-méthanol (80-20 en volumes)] et concentration à sec des fractions 12 à 22 sous pression réduite (2,7 kPa) à 30 °C, on obtient 1,05 g d'iodure de (méthyl-2 triméthylammonio-3 propyl) thiométhylène-5δ pristinamycine $I_A$ sous forme d'une poudre ocre fondant vers 150 °C.

Spectre RMN :

1,05 - 1,35 (m, 8H : $1\gamma + 3\gamma_2 + 3\beta + -\underset{|}{C}H-C\underline{H}_3$)

2,40 (m, 2H : $5\beta_1 + -\underset{|}{C\underline{H}}-CH_3$)

2,90 (mt, 3H : $4\beta_2 + -S-C\underline{H}_2-$)

3,20 (mt, 7H : $4NCH_3 + 4\beta_1 + 3\delta_1 + -C\underline{H}_2\overset{\oplus}{N}(CH_3)_3$)

3,40 (mt, 9H : $-\overset{\oplus}{N}(C\underline{H}_3)_3$)

On obtient une solution aqueuse à 1 % de (méthyl-2 triméthylammonio-3 propyl) thiométhylène-5δ pristinamycine $I_A$, iodure, (produit AAL), à l'état de chlorhydrate, avec :

| | |
|---|---|
| produit AAL | 0,02 g |
| acide chlorhydrique 0,1N | 0,2 $cm^3$ |
| eau distillée | qsp 2 $cm^3$ |

L'iodure de mercapto-3 méthyl-2 propylammonium peut être préparé de la manière suivante : on ajoute 0,024 g de méthylate de sodium à une solution de 3,6 g d'iodure d'acétylthio-3 méthyl-2 propylammonium dans 18 $cm^3$ de méthanol à une température voisine de 20 °C. Le mélange obtenu est porté au reflux pendant 1 heure puis laissé à température ambiante pendant 16 heures. Le méthanol est éliminé sous pression réduite (2,7 kPa) à 50 °C. Le résidu est agité pendant 1 heure avec 35 $cm^3$ d'isopropanol et la suspension blanche est filtrée puis séchée. On obtient ainsi 3,1 g d'iodure de mercapto-3 méthyl-2 propylammonium sous forme d'une poudre beige fondant à 120 °C.

L'iodure d'acétylthio-3 méthyl-2 propylammonium peut être préparé de la manière suivante : on ajoute 1,4 $cm^3$ d'iodure de méthyle à une solution de 3,5 g de N,N-diméthyl acétylthio-3 méthyl-2 propylamine dans 35 $cm^3$ d'acétonitrile ; après 18 heures d'agitation à une température voisine de 20 °C, le précipité est filtré puis séché. On obtient ainsi 3,8 g d'iodure d'acétylthio-3 méthyl-2 propylammonium sous forme d'une poudre blanche fondant à 181 °C.

Exemple 43

En opérant d'une manière analogue à celle décrite à l'exemple 28, mais à partir de 1,84 g de diméthylaminométhylène-5δ pristinamycine $I_A$ et 3,28 g de sel de sodium de l'acide mercapto-2 éthanesulfonique et après purification par chromatographie « flash » [éluant : chlorure de méthylène-méthanol (90-10 en volumes)] et concentration à sec des fractions 6 à 14 sous pression réduite (2,7 kPa) à 30 °C, on obtient 0,8 g d'(hydroxysulfonyl-2 éthyl) thiométhylène-5δ pristinamycine $I_A$ sous forme d'une poudre jaune fondant à une température supérieure à 280 °C.

Le spectre infra-rouge comporte les bandes caractéristiques des pristinamycines : 1 745, 1 680, 1 650, 1 525, 815, 740 et 705 $cm^{-1}$, plus les bandes caractéristiques du groupe $—SO_3H$ [1 200 $cm^{-1}$ (large) et 1 050 $cm^{-1}$].

On obtient une solution aqueuse à 5 % d'(hydroxysulfonyl-2 éthyl) thiométhylène-5δ pristinamycine $I_A$ (produit AAM), avec :

| | |
|---|---|
| produit AAM | 0,1 g |
| eau distillée | qsp 2 $cm^3$ |

Exemple 44

A une solution de 0,87 g de (mercapto-2 propyl)-1 méthyl-4 pipérazine dans 50 $cm^3$ d'éthanol additionnée de 0,34 g d'éthylate de sodium, on ajoute une solution de 5,2 g de (méthyl-4 phényl) sulfonyloxyméthylène-5δ pristinamycine $I_A$ dans 50 $cm^3$ de chlorure de méthylène. Le mélange réactionnel est agité pendant 16 heures à une température voisine de 20 °C puis dilué avec 500 $cm^3$ de chlorure de méthylène et 100 $cm^3$ d'eau distillée. Après agitation, la phase aqueuse est extraite 2 fois par 50 $cm^3$ de chlorure de méthylène au total. Les phases organiques sont réunies, séchées sur du sulfate de magnésium, filtrées puis concentrées à sec sous pression réduite (2,7 kPa) à 30 °C. Le résidu est purifié par chromatographie « flash » [éluant : chloroforme-méthanol (97,5-2,5 en volumes)]. Les fractions 33 à 80 sont réunies et concentrées à sec sous pression réduite (2,7 kPa) à 30 °C. On obtient ainsi 1,25 g de [(méthyl-4 pipérazinyl-1)-3 propyl-2] thiométhylène-5δ pristinamycine $I_A$ sous forme d'une poudre beige fondant vers 195 °C.

Spectre RMN :

0,70 (dd, 1H : $5\beta_2$)

1,25 (d, 3H : $-CH-\underline{CH}_3$)

2,30 (s, 3H : $>N-\underline{CH}_3$)

2,50 (m, 10H : 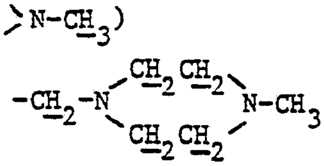 )

3,40 (dd, 1H : $5\varepsilon_2$)

7,85 (dd large, 1H : $1'H_6$)

On obtient une solution aqueuse à 10 % de [(méthyl-4 pipérazinyl-1)-3 propyl-2] thiométhylène-5δ pristinamycine $I_A$ (produit AAN) sous forme de chlorhydrate, avec :

| | |
|---|---|
| produit AAN | 0,03 g |
| acide chlorhydrique 0,1N | 0,3 $cm^3$ |

La (mercapto-2 propyl)-1 méthyl-4 pipérazine est préparée en chauffant à 100 °C pendant 16 heures un mélange de 19 $cm^3$ de sulfure de propylène et de 29 $cm^3$ de N-méthylpipérazine. On obtient ainsi 32 g d'une huile incolore distillant à 105 °C sous 1,3 kPa.

La (méthyl-4 phényl) sulfonyloxyméthylène-5δ pristinamycine $I_A$ peut être obtenue de la manière suivante :

A une solution de 2,7 g d'hydroxyméthylène-5δ pristinamycine $I_A$ dans 30 $cm^3$ de chlorure de méthylène, on ajoute à une température voisine de —30 °C, 0,42 $cm^3$ de triéthylamine puis 0,57 g de chlorure de l'acide p-toluènesulfonique. Le mélange réactionnel est agité ensuite pendant 2 heures à une température voisine de 20 °C, puis concentré à sec sous pression réduite (2,7 kPa) à 30 °C ; le résidu obtenu est purifié par chromatographie « flash » [éluant : chlorure de méthylène-méthanol (96-4 en volumes)]. Après concentration à sec des fractions 4 à 6 sous pression réduite (2,7 kPa) à 30 °C, on obtient 2,2 g de (méthyl-4 phényl) sulfonyloxyméthylène-5δ pristinamycine $I_A$ sous forme d'une poudre blanche fondant vers 265 °C.

Spectre RMN :

0,50 (dd, 1H : $5\beta_2$)

2,35 (s, 3H : $-SO_2-$ 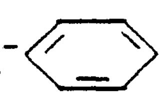 $-\underline{CH}_3$ )

3,30 (dd, 1H : 5$\varepsilon_2$)
5,25 (d, 1H : 5α)
5,30 (dd, 1H : 5$\varepsilon_1$)
7,35 à 7,90 (système AB + m, 8H : 4δ+ 4ε +

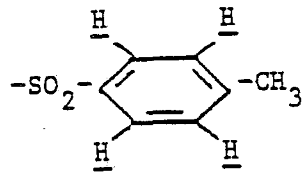 )

7,85 (dd, 1H : 1'$H_6$)

L'hydroxyméthylène-5δ pristinamycine $I_A$ peut être préparée de la manière suivante :

A 420 $cm^3$ d'une solution aqueuse 0,1N d'acide chlorhydrique, on ajoute sous agitation 10,6 g de diméthylaminométhylène-5δ pristinamycine $I_A$. La solution obtenue est ensuite agitée pendant 3 heures à une température voisine de 20 °C. On ajoute alors goutte à goutte 30 $cm^3$ d'une solution aqueuse saturée de bicarbonate de sodium de manière à obtenir un pH voisin de 4. Le produit qui précipite est séparé par filtration puis lavé 3 fois par 30 $cm^3$ au total d'eau distillée. Après séchage sous pression réduite (2,7 kPa) à une température voisine de 20 °C, on obtient 9,5 g d'hydroxyméthylène-5δ pristinamycine $I_A$ sous forme d'une poudre beige. Ce produit est d'une qualité suffisante pour être utilisé tel quel dans les phases ultérieures. Il peut toutefois être purifié de la manière suivante :

9,5 g d'hydroxyméthylène-5δ pristinamycine $I_A$ brute sont dissous dans 50 $cm^3$ d'acétate d'éthyle ; la solution obtenue est versée sur 100 g de gel de silice contenus dans une colonne de 2,8 cm de diamètre. On élue d'abord avec 400 $cm^3$ d'acétate d'éthyle et élimine l'éluat correspondant ; on élue ensuite avec 1 600 $cm^3$ d'acétate d'éthyle et concentre l'éluat correspondant à sec sous pression réduite (2,7 kPa) à 30 °C. On obtient ainsi 6,3 g d'hydroxyméthylène-5δ pristinamycine $I_A$ sous forme de cristaux blancs fondant à 220 °C.

Spectre RMN :

0,69 (dd, 1H : 5$\beta_2$)
2,43 (d, 1H : 5$\beta_1$)
3,40 (d, 1H : 5$\varepsilon_2$)
4,0 à 4,2 (m, 3H : 4α + 5$\varepsilon_1$ + 5α)
8,15 (s, 1H : =C<u>H</u>-OH)
11,63 (s large, 1H : =CH-O<u>H</u>)

## Exemple 45

En opérant d'une manière analogue à celle décrite à l'exemple 44, mais à partir de 5,2 g de (méthyl-4 phényl) sulfonyloxyméthylène-5δ pristinamycine $I_A$, 0,6 g de diméthylamino-1 propanethiol-2 et 0,34 g d'éthylate de sodium et après purification par chromatographie « flash » [éluant : chloroforme-méthanol (95-5 en volumes)] et concentration à sec des fractions 16 à 38 sous pression réduite (2,7 kPa) à 30 °C, on obtient 1 g de (diméthylamino-3 propyl-2) thiométhylène-5δ pristinamycine $I_A$ sous forme d'une poudre jaune fondant à 172 °C.

Spectre RMN :

0,65 (dd, 1H : 5$\beta_2$)
1,10 (d, 3H : -CH-C<u>H</u>$_3$)
2,30 (s, 6H : -N(C<u>H</u>$_3$)$_2$)
7,60 (s large, 1H : =C<u>H</u>-S-)
7,85 (dd, 1H : 1'$H_6$)

On obtient une solution aqueuse à 5 % de (diméthylamino-3 propyl-2) thiométhylène-5δ pristinamycine $I_A$ (produit AAO), à l'état de chlorhydrate, avec :

| | |
|---|---|
| produit AAO | 0,03 g |
| acide chlorhydrique 0,1N | 0,3 cm³ |
| eau distillée | qsp 0,6 cm³ |

Le diméthylamino-1 propanethiol-2 peut être préparé selon la méthode décrite par S. D. Turk et coll., J. Org. Chem. *29*, 974 (1964).

Exemple 46

En opérant d'une manière analogue à celle décrite à l'exemple 44, mais à partir de 6,3 g de (méthyl-4 phényl) sulfonyloxyméthylène-5δ pristinamycine $I_A$, de 1,05 g de diéthylamino-5 pentanethiol-2 et 0,408 g d'éthylate de sodium et après purification par chromatographie « flash » [éluant : chloroforme-méthanol (97,5-2,5 en volumes)] et concentration à sec des fractions 47 à 65 sous pression réduite (2,7 kPa) à 30 °C, on obtient 1,32 g de (diéthylamino-5 pentyl-2) thiométhylène-5δ pristinamycine $I_A$ sous forme d'une poudre beige fondant vers 185 °C.

Spectre RMN :

0,65 (dd, 1H : $5\beta_2$)
1,20 (t, 6H : $-N(CH_2C\underline{H}_3)_2$)
1,40 (d, 3H : $-CH-C\underline{H}_3$)
1,70 (s large, 4H : $-CH(C\underline{H}_2)_2-CH_2N\langle$)
2,65 (q, 4H : $-N(C\underline{H}_2-CH_3)_2$)
3,50 (dd, 1H : $5\varepsilon_2$)
7,65 (s large, 1H : $=C\underline{H}-S-$)
7,85 (dd, 1H : $1'H_6$)

On obtient une solution aqueuse à 10 % de (diéthylamino-5 pentyl-2) thiométhylène-5δ pristinamycine $I_A$ (produit AAP), sous forme de chlorhydrate, avec :

| | |
|---|---|
| produit AAP | 0,05 g |
| acide chlorhydrique 0,1N | 0,5 cm³ |

Le diéthylamino-5 pentanethiol-2 peut être préparé d'une manière analogue à celle décrite à l'exemple 32 pour préparer le diméthylamino-3 méthyl-2 propanethiol, mais à partir de 4,0 g de N,N-diéthyl acétylthio-4 pentanamine-1 et 0,046 g de sodium. Après purification par chromatographie « flash » [éluant : acétate d'éthyleméthanol 70-30 en volumes)] et concentration à sec des fractions 16 à 24, on obtient 2,0 g de diéthylamino-5 pentanethiol-2 sous forme d'une huile jaune.

La N,N-diéthyl acétylthio-4 pentanamine-1 peut être préparée d'une manière analogue à celle décrite dans l'exemple 32 pour préparer la N,N-diméthyl acétylthio-3 méthyl-2 propylamine, mais à partir de 32 g de N,N-diéthyl chloro-4 pentanamine-1 et 15,2 g d'acide thiolacétique. On obtient ainsi 4,31 g de produit sous forme d'une huile jaune.

La N,N-diéthylchloro-4 pentanamine-1 peut être préparée selon la méthode décrite par M. S. Kharash et C. F. Fuchs, brevet US 2 432 905.

Exemple 47

Une solution de 7,6 g de [(méthyl-4 phényl) sulfonyloxyméthylène]-5δ pristinamycine $I_A$ dans 60 cm³ de tétrahydrofuranne est refroidie à une température voisine de — 10 °C. On y ajoute lentement en maintenant cette température une solution de 0,65 g de diméthylamino-2 éthanol dans 60 cm³ de tétrahydrofuranne additionnée de 0,35 g d'une dispersion à 50 % d'hydrure de sodium dans l'huile minérale. A la fin de l'addition on laisse remonter lentement la température au voisinage de 20 °C. Le mélange réactionnel est agité pendant 24 heures à cette température puis dilué avec 500 cm³ de chlorure de méthylène et lavé 2 fois avec 50 cm³ d'une solution saturée de chlorure d'ammonium. La phase organique est séchée sur du sulfate de magnésium, filtrée puis concentrée à sec sous pression réduite (2,7 kPa) à 40 °C. Le résidu obtenu est purifié par chromatographie « flash » [éluant : chloroforme-méthanol (95-5 en volumes)]. Les fractions 12 à 17 sont réunies et concentrées à sec sous pression réduite (2,7 kPa) à 25 °C. On obtient ainsi 1,5 g de (diméthylamino-2 éthoxyméthylène)-5δ pristinamycine $I_A$ sous forme d'une poudre beige fondant vers 160 °C.

Spectre RMN :

0,65 (dd, 1H : 5$\beta_2$)
2,3 (s, 6H : $-N(\underline{CH_3})_2$)
2,65 (m, 2H : $-\underline{CH_2}N<$)
3,42 (dd, 1H : 5$\varepsilon_2$)
4,15 (t, 2H : $-O\underline{CH_2}-$)
5,15 (d, 1H : 5$\varepsilon_1$)
7,45 (sous les aromatiques, 1H : $>C=C\underline{H}O-$)
7,80 (dd, 1H : 1'$H_6$)

On obtient une solution aqueuse à 1 % de (diméthylamino-2 éthoxyméthylène)-5δ pristinamycine $I_A$ (produit AAQ), sous forme de chlorhydrate, avec :

| | |
|---|---|
| produit AAQ | 0,03 g |
| acide chlorhydrique 0,1N | 0,3 cm³ |
| eau distillée | qsp 3 cm³ |

## Exemple 48

A une solution de 0,5 g de (méthyl-4 phényl) sulfonyloxy méthylène-5δ pristinamycine $I_A$ dans 25 cm³ d'éthanol, on ajoute 0,12 g d'amino-4 méthyl-1 pipéridine à une température voisine de 20 °C. Après 16 heures d'agitation à cette température, le mélange réactionnel est dilué avec 100 cm³ de chlorure de méthylène, lavé deux fois avec 100 cm³ au total d'eau distillée. La phase organique est séchée sur sulfate de sodium puis concentrée sous pression réduite (2,7 kPa) à 30 °C. Le résidu est agité avec 15 cm³ d'éther éthylique. Après filtration, on obtient 0,42 g de (méthyl-1 pipéridyl-4) aminométhylène-5δ pristinamycine $I_A$ sous forme d'une poudre blanche dont les caractéristiques sont identiques à celles décrites à l'exemple 24.

## Exemple 49

En opérant de manière analogue à ceux décrits dans les exemples 11 et 23, à partir d'un produit de formule générale (IV) ou de formule générale (IX), on prépare les dérivés de synergistine de formule générale (I) suivants :

| Produit de départ [Y = $N(CH_3)_2$] ($-NR_1R_2$ ou $-Z$) | Conditions de la réaction | Produit défini précédemment à l'exemple N° |
|---|---|---|
| $-NR_1R_2$ = $-NH_2$ | $CH_3COOH$, 20°C, 20 heures | 24 |
| $-NR_1R_2$ = $-NH\ C_{10}H_{21}$ | $CH_3COOH$, $CF_3COOH$, 20°C, 20 heures | 30 |
| $-NR_1R_2$ = -N 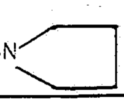 | $CH_3COOH$, 20°C, 20 heures | 24 |
| $-NR_1R_2$ = $-NH-C_6H_5$ | $CH_3COOH$, $CF_3COOH$, 20°C, 48 heures | 24 |

(Suite)

| Produit de départ [Y = $N(CH_3)_2$] ($-NR_1R_2$ ou $-Z$ ) | Conditions de la réaction | Produit défini précédemment à l'exemple N° |
|---|---|---|
| $-NR_1R_2$ = $-NH(CH_2)_3$ $NH(CH_2)_2OH$ | $CH_3COOH$, $CF_3COOH$, 20°C, 20 heures | 30 |
| $-NR_1R_2$ = $-NH(CH_2)_3N[(CH_2)_2OH]_2$ | $CH_3COOH$, $CF_3COOH$, 20°C, 48 heures | 30 |
| $-NR_1R_2$ = $-NH(CH_2)_2SH$ | $CH_3COOH$, 20°C, 20 heures | 30 |
| $-NR_1R_2$ = $-NH(CH_2)_2NHC_6H_5$ | $CH_3COOH$, 20°C, 10 jours | 24 |
| $-NR_1R_2$ = $-NHCH_2-$ 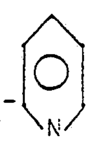 | $CH_3COOH$, $CF_3COOH$, 20°C, 20 heures | 30 |
| Z = $-OCOCH_3$ | $CH_3COOH$, 20°C, 6 heures | 24 |
| Z = $-OPO(OC_2H_5)_2$ | $CH_3COOH$, 20°C, 20 heures | 24 |

Les produits de formule générale (IV) mis en œuvre peuvent être préparés comme décrit ci-après ou par analogie avec cette méthode.

A une solution de 1,84 g de diméthylaminométhylène-5δ pristinamycine $I_A$ dans 20 $cm^3$ d'acide acétique, on ajoute lentement 10 $cm^3$ d'une solution éthanolique 4N d'ammoniac gazeux. La solution obtenue est agitée pendant 20 heures à une température voisine de 20 °C, puis est versée lentement dans 200 $cm^3$ d'une solution aqueuse saturée de bicarbonate de sodium. La suspension obtenue est extraite 3 fois par 300 $cm^3$ au total de chlorure de méthylène ; les phases organiques sont réunies, séchées sur du sulfate de magnésium, filtrées et concentrées à sec sous pression réduite (2,7 kPa) à 30 °C. Le résidu obtenu est purifié par chromatographie « flash » [éluant : chloroforme-méthanol (92-8 en volumes)]. Par concentration à sec des fractions 11 à 13 sous pression réduite (2,7 kPa) à 30 °C, on obtient 1,3 g d'aminométhylène-5 pristinamycine $I_A$ sous forme d'une poudre beige fondant vers 190 °C.

Spectre RMN :

0,7 à 1,10 (mt, 4H : $2\gamma + 5\beta_2$)
7,15 à 7,53 (mt, 9H (dont 1 échangeable) : $6\gamma + 6\delta + 6\varepsilon$ + 1H du $NH_2$ + = $C\underline{H}-NH_2$ + $l'H_4$ + $l'H_5$)
9,12 (s large, 1H (échangeable) : 1H du $NH_2$).

Les produits de formule générale (IX) mis en œuvre peuvent être préparés de la manière suivante :

— acétoxyméthylène-5δ pristinamycine $I_A$ :

A une solution de 1,8 g d'hydroxyméthylène-5δ pristinamycine $I_A$ dans 20 $cm^3$ de chlorure de méthylène contenant 0,2 g de triéthylamine, on ajoute à une température voisine de — 20 °C 0,14 $cm^3$ de chlorure d'acétyle puis on laisse remonter la température à environ 20 °C.

Le mélange réactionnel est agité ensuite pendant 20 heures à cette température, puis concentré à sec sous pression réduite (2,7 kPa) à 30 °C ; le résidu obtenu est purifié par chromatographie « flash » [éluant : acétate d'éthyle]. Après concentration à sec des fractions 4 à 7 sous pression réduite (2,7 kPa) à 30 °C, on obtient 0,7 g d'acétoxyméthylène-5δ pristinamycine $I_A$ sous forme d'une poudre jaune fondant vers 160 °C.

Spectre RMN :

0,60 (dd; 1H : 5$\beta_2$)

2,25 (s; 3H : $-CO-C\underline{H}_3$)

2,45 (d; 1H : 5$\beta_1$)

3,45 (dd; 1H : 5$\varepsilon_2$)

5,25 (dd; 1H : 5$\alpha$)

5,45 (d; 1H : 5$\varepsilon_1$)

7,10 à 7,45 (m; 8H : 6$\gamma$ + 6$\delta$ + 6$\varepsilon$ + 1'$H_4$ + 1'$H_5$ + $=C\underline{H}-O-$)

7,85 (dd; 1H : 1'$H_6$)

— diéthoxyphosphoryloxyméthylène-5δ pristinamycine $I_A$ :

En opérant d'une manière analogue à celle décrite ci-dessus, mais à partir de 1,8 g d'hydroxyméthylène-5δ pristinamycine $I_A$ et 0,34 g de chlorophosphate de diéthyle et après purification par chromatographie « flash » [éluant : acétate d'éthyle-méthanol (90-10 en volumes)] et concentration à sec des fractions 6 à 14 sous pression réduite (2,7 kPa) à 30 °C, on obtient 0,8 g de diéthoxyphosphoryloxyméthylène-5δ pristinamycine $I_A$ sous forme d'une poudre jaune fondant vers 150 °C.

Spectre RMN :

0,55 (dd; 1H : 5$\beta_2$)

1,30 (td; 6H : $-PO(O-CH_2-C\underline{H}_3)_2$)

2,40 (d; 1H : 5$\beta_1$)

3,40 (dd; 1H : 5$\varepsilon_2$)

4,25 (ddd; 4H : $-PO(O-C\underline{H}_2-CH_3)_2$

5,25 (d; 1H : 5$\alpha$ )

5,40 (d; 1H : 5$\varepsilon_1$)

7,10 à 7,55 (m; 8H : 6$\gamma$ + 6$\delta$ + 6$\varepsilon$ + $=C\underline{H}-O-$ + 1'$H_5$ + 1'$H_4$)

7,85 (dd; 1H x 0,85 : 1'$H_6$ 1er isomère)

8 (dd; 1H x 0,15 : 1'$H_5$ 2eme isomère)

L'hydroxyméthylène-5δ pristinamycine $I_A$ peut être préparée comme décrit à l'exemple 44.

## Exemple 50

En opérant de manière analogue à la méthode décrite à l'exemple 48, mais en agitant pendant 20 heures, on prépare la (diméthylamino-3 propyl) thiométhylène-5δ pristinamycine $I_A$ décrite précédemment à l'exemple 30, à partir de la chlorométhylène-5δ pristinamycine $I_A$.

Le produit de départ peut être préparé de la manière suivante :

Dans une solution de 1,3 $cm^3$ de triphénylphosphite dans 25 $cm^3$ de chlorure de méthylène, on fait passer un courant de chlore gazeux jusqu'à obtenir une couleur jaune-vert persistante, en maintenant la température entre — 20 °C et — 15 °C. On ajoute ensuite 6 gouttes de triphénylphosphite pour décolorer la solution, puis 4,1 g d'hydroxyméthylène-5δ pristinamycine $I_A$, toujours en maintenant la température entre — 20 °C et — 15 °C. On agite la solution obtenue pendant 1 heure à — 15 °C, puis on ajoute goutte à goutte une solution de 0,4 $cm^3$ de pyridine dans 25 $cm^3$ de chlorure de méthylène. On agite ensuite le mélange réactionnel 30 minutes à une température voisine de 20 °C, puis on ajoute 0,46 $cm^3$ d'acide chlorhydrique concentré (d = 1,19) et 50 $cm^3$ de chlorure de méthylène. Le mélange est lavé 4 fois par 100 $cm^3$ au total d'eau distillée ; la phase organique est séchée sur du sulfate de magnésium, filtrée puis concentrée à sec sous pression réduite (2,7 kPa) à 30 °C. Le résidu obtenu est purifié par chromatographie « flash » [éluant : acétate d'éthyle] ; après concentration à sec des fractions 7 à 9 sous pression réduite (2,7 kPa) à 30 °C, on obtient 1,2 g de chlorométhylène-5δ pristinamycine $I_A$ sous forme d'une poudre beige fondant vers 190 °C.

Spectre RMN :

0,55 (dd; 1H : $5\beta_2$)
2,45 (d; 1H : $5\beta_1$)
3,45 (dd; 1H : $5\varepsilon_2$)
5,30 (d; 1H : $5\alpha$)
5,45 (d; 1H : $5\varepsilon_1$)
7,15 à 7,60 (m; 8 H : $6\gamma + 6\delta + 6\varepsilon + 1'H_4 + 1'H_5 +$ =C<u>H</u>-Cl)
7,85 (dd; 1H : $1'H_6$)

La présente invention concerne également les médicaments constitués par un produit de formule générale (I), sous forme libre ou sous forme de sel d'addition avec un acide ou le cas échéant une base pharmaceutiquement acceptable ; à l'état pur ou sous forme d'une composition dans laquelle il est associé à tout diluant ou adjuvant pharmaceutiquement compatible. Les médicaments selon l'invention peuvent être utilisés par voie parentérale, orale, rectale ou topique.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement des infections d'origine bactérienne. Les doses dépendent de l'effet recherché et la durée du traitement. Pour un adulte, elles sont généralement comprises entre 2 000 et 4 000 mg par jour par voie parentérale, particulièrement par voie intraveineuse en perfusion lente.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

### Exemple A

On prépare, selon la technique habituelle, une solution injectable pour perfusion contenant 5 g/l de produit actif ayant la composition suivante :

| | |
|---|---|
| — (diméthylamino-3 propyl) thiométhyl-5δ pristinamycine $I_A$ | 5 g |
| — solution aqueuse d'acide chlorhydrique 0,1N | 50 $cm^3$ |
| — eau distillée | qsp 1 000 $cm^3$ |

### Exemple B

On prépare, selon la technique habituelle, une solution injectable pour perfusion contenant 5 g/l de produit actif ayant la composition suivante :

| | |
|---|---|
| — [(méthyl-4 pipérazinyl-1)-2 éthyl] thiométhyl-5δ pristinamycine $I_A$ | 5 g |
| — solution aqueuse d'acide chlorhydrique 0,1N | 48 $cm^3$ |
| — eau distillée | qsp 1 000 $cm^3$ |

**Revendications** (pour les Etats contractants : BE, CH, DE, FR, GB, IT, LI, LU, NL, SE)

1. Nouveau dérivé de synergistines caractérisé en ce qu'il répond à la formule générale :

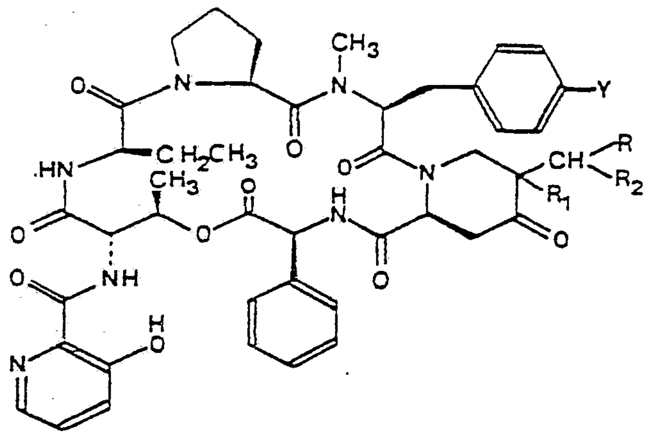

dans laquelle Y représente un atome d'hydrogène ou un radical diméthylamino et

a) soit $R_1$ et $R_2$ représentent chacun un atome d'hydrogène et R représente un radical pyrrolidinyl-3 thio ou pipéridyl-3 ou 4 thio (ces radicaux étant éventuellement substitués par un radical alcoyle) ou bien R représente un radical alcoylthio substitué par un ou deux radicaux hydroxysulfonyle, alcoylamino, dialcoylamino (éventuellement substitué par un radical mercapto ou dialcoylamino) ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle),

b) soit $R_1$ et $R_2$ forment ensemble une liaison de valence et R représente un radical pyrrolidinyl-3 amino, pipéridyl-3 ou 4 amino, pyrrolidinyl-3 oxy, pipéridyl-3 ou 4 oxy, pyrrolidinyl-3 thio, pipéridyl-3 ou 4 thio (ces radicaux étant éventuellement substitués sur l'atome d'azote du cycle par un radical alcoyle), ou bien R représente un radical alcoylamino, alcoyloxy ou alcoylthio substitués par un ou deux radicaux hydroxysulfonyle, alcoylamino, dialcoylamino (éventuellement substitué par un radical dialcoylamino), trialcoylammonio ou imidazolyle-4 ou 5 ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle), étant entendu que les radicaux alcoyle et portions alcoyle contiennent 1 à 5 atomes de carbone en chaîne droite ou ramifiée, sous ses formes isomères lorsqu'elles existent et leurs mélanges, ainsi que leurs sels pharmaceutiquement acceptables.

2. Procédé de préparation d'un produit selon la revendication 1 dans la formule duquel Y est défini comme à la revendication 1 et les autres symboles sont définis comme à la revendication 1 en a), caractérisé en ce qu'on fait réagir un produit de formule générale :

$$R'—H$$

dans laquelle R' a la définition de R donnée en a) à la revendication 1, sur un produit de formule générale :

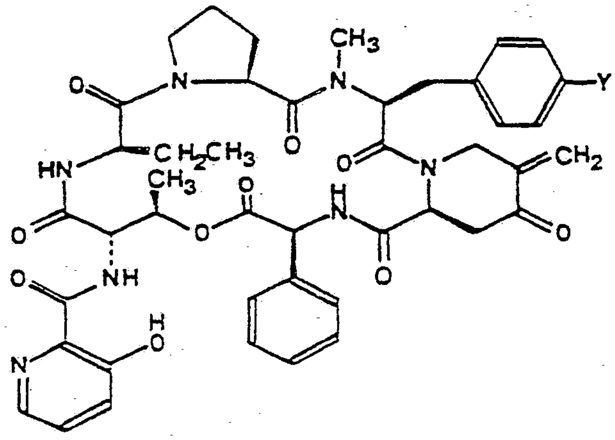

dans laquelle Y est défini comme à la revendication 1, puis isole le produit et le transforme éventuellement en un sel pharmaceutiquement acceptable.

3. Procédé de préparation d'un produit selon la revendication 1 dans la formule duquel Y est défini comme à la revendication 1 et les autres symboles sont définis comme à la revendication 1 en b) à l'exception pour R de représenter un radical pyrrolidinyl-3 oxy, pipéridyl-3 ou 4 oxy ou alcoyloxy (éventuellement substitué comme défini en b) à la revendication 1), caractérisé en ce que l'on fait réagir un produit de formule générale :

R″—H

dans laquelle R″ a la définition de R donnée à la revendication 1 en b) à l'exception de représenter un radical pyrrolidinyl-3 oxy, pipéridyl-3 ou 4 oxy ou alcoyloxy (éventuellement substitués comme défini en b) à la revendication 1) sur un produit de formule générale :

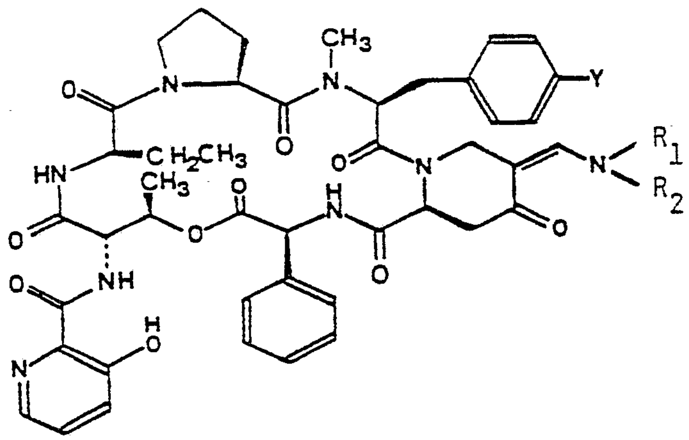

dans laquelle Y est défini comme à la revendication 1, et $R_1$ et $R_2$, identiques ou différents, représentent un atome d'hydrogène ou un radical phényle ou pyridyle éventuellement substitués (par un radical dialcoylamino dont la partie alcoyle contient 1 à 4 atomes de carbone en chaîne droite ou ramifiée) ou un radical alcoyle contenant 1 à 10 atomes de carbone en chaîne droite ou ramifiée éventuellement substitués [par un radical hydroxy, mercapto carboxy, pyridyle, anilino, alcoylamino ou dialcoylamino dont au moins l'une des parties alcoyle est elle-même substituée par un radical hydroxy, mercapto, carboxy ou anilino], ou un radical alcényle contenant 3 ou 4 atomes de carbone, ou un radical alcynyle contenant 3 ou 4 atomes de carbone, ou bien $R_1$ et $R_2$ forment ensemble avec l'atome d'azote auquel ils sont liés un hétérocycle à 5 ou 6 chaînons contenant éventuellement un autre hétéroatome choisi parmi l'oxygène, le soufre ou l'azote (éventuellement substitué par un radical alcoyle), puis lorsqu'ils existent sépare éventuellement les isomères du produit obtenu, et transforme éventuellement le produit obtenu en un sel pharmaceutiquement acceptable.

4. Procédé de préparation d'un produit selon la revendication 1 dans la formule duquel Y est défini comme à la revendication 1 et les autres symboles sont définis comme à la revendication 1 en b), caractérisé en ce que l'on fait réagir un produit de formule générale :

R‴—H

dans laquelle R‴ a la définition de R donnée en b) à la revendication 1, sur un produit de formule générale :

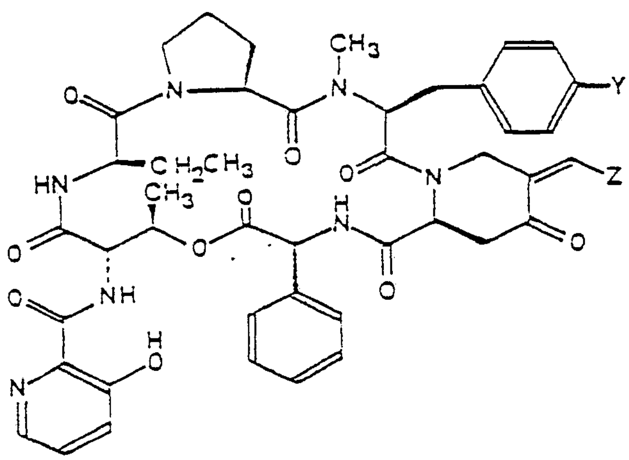

dans laquelle Y est défini comme à la revendication 1 et Z représente un atome d'halogène, un radical triméthylsilyloxy, dialcoyloxyphosphoryloxy ou un radical de formule générale :

$$-OSO_2R_3$$

ou

$$-OCOR_4$$

dans lesquelles $R_3$ est un radical alcoyle, trifluorométhyle, trichlorométhyle ou un radical phényle éventuellement substitué par un atome d'halogène ou par un radical alcoyle ou nitro et $R_4$ est défini comme $R_3$ ou représente un radical alcoylcarbonylméthyle, alcoylcarbonyléthyle, alcoyloxycarbonylméthyle, alcoyloxycarbonyl-2 éthyle ou alcoyloxy, puis lorsqu'ils existent sépare éventuellement les isomères du produit obtenu, et transforme éventuellement le produit obtenu en un sel pharmaceutiquement acceptable.

5. Composition pharmaceutique caractérisée en ce qu'elle contient au moins un dérivé selon la revendication 1, en association avec un ou plusieurs diluants ou adjuvants compatibles et pharmaceutiquement acceptables.

**Revendication** (pour l'Etat contractant AT)

Un procédé de préparation de nouveaux dérivés de synergistines de formule générale :

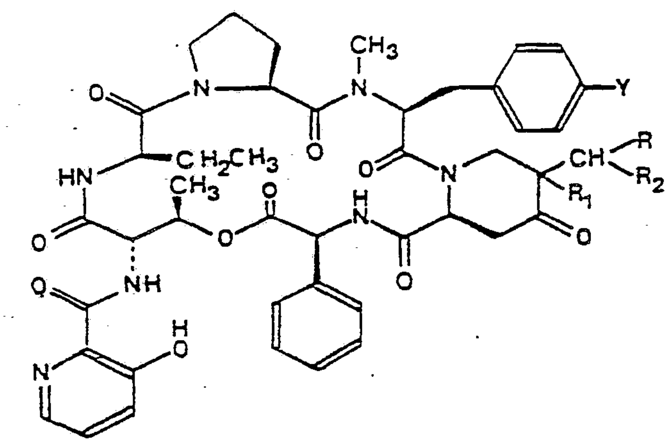

(I)

dans laquelle Y représente un atome d'hydrogène ou un radical diméthylamino et

a) soit $R_1$ et $R_2$ représentent chacun un atome d'hydrogène et R représente un radical pyrrolidinyl-3 thio ou pipéridyl-3 ou 4 thio (ces radicaux étant éventuellement substitués par un radical alcoyle) ou bien R représente un radical alcoylthio substitué par un ou deux radicaux hydroxysulfonyle, alcoylamino, dialcoylamino (éventuellement substitué par un radical mercapto ou dialcoylamino) ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle),

b) soit $R_1$ et $R_2$ forment ensemble une liaison de valence et R représente un radical pyrrolidinyl-3 amino, pipéridyl-3 ou 4 amino, pyrrolidinyl-3 oxy, pipéridyl-3 ou 4 oxy, pyrrolidinyl-3 thio, pipéridyl-3 ou 4 thio (ces radicaux étant éventuellement substitués sur l'atome d'azote du cycle par un radical alcoyle), ou bien R représente un radical alcoylamino, alcoyloxy ou alcoylthio substitués par un ou deux radicaux hydroxysulfonyle, alcoylamino, dialcoylamino (éventuellement substitué par un radical dialcoylamino), trialcoylammonio ou imidazolyle-4 ou 5 ou par un ou deux cycles choisis parmi pipérazino (éventuellement substitué par un radical alcoyle ou mercaptoalcoyle), morpholino, thiomorpholino, pipéridino, pyrrolidinyle-1, pipéridyle-2, 3 ou 4 et pyrrolidinyle-2 ou 3 (ces deux derniers cycles étant éventuellement substitués sur l'atome d'azote par un radical alcoyle), étant entendu que les radicaux alcoyle et portions alcoyle contiennent 1 à 5 atomes de carbone en chaîne droite ou ramifiée, sous leurs formes isomères lorsqu'elles existent et leurs mélanges, ainsi que leurs sels pharmaceutiquement acceptables, caractérisé en ce que lorsque Y est défini comme précédemment et les autres symboles sont définis comme ci-dessus en a), on fait réagir un produit de formule générale :

$$R'-H$$

dans laquelle R' a la définition de R donnée précédemment en a) sur un produit de formule générale :

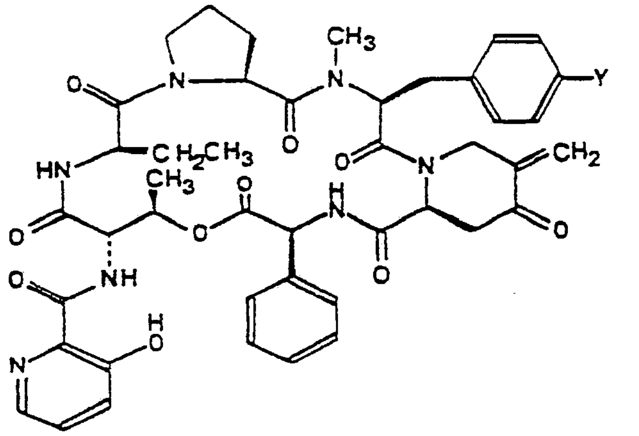

dans laquelle Y est défini comme précédemment, ou bien lorsque Y est défini comme précédemment et les autres symboles sont définis comme précédemment en b) à l'exception pour R de représenter un radical pyrrolidinyl-3 oxy, pipéridyl-3 ou 4 oxy ou alcoyloxy (éventuellement substitué comme défini précédemment en b), caractérisé en ce que l'on fait réagir un produit de formule générale :

R"—H

dans laquelle R" a la définition de R donnée précédemment en b) à l'exception de représenter un radical pyrrolidinyl-3 oxy, pipéridyl-3 ou 4 oxy ou alcoyloxy (éventuellement substitués comme défini en b) sur un produit de formule générale :

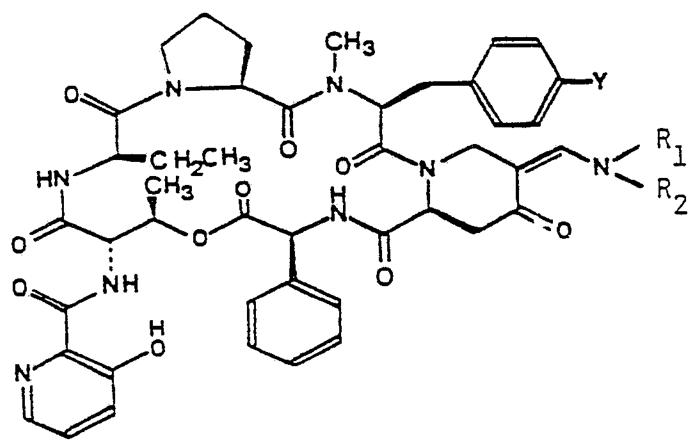

dans laquelle Y est défini comme précédemment et $R_1$ et $R_2$, identiques ou différents, représentent un atome d'hydrogène ou un radical phényle ou pyridyle éventuellement substitués (par un radical dialcoylamino dont la partie alcoyle contient 1 à 4 atomes de carbone en chaîne droite ou ramifiée) ou un radical alcoyle contenant 1 à 10 atomes de carbone en chaîne droite ou ramifiée éventuellement substitués [par un radical hydroxy, mercapto, carboxy, pyridyle, anilino, alcoylamino ou dialcoylamino dont au moins l'une des parties alcoyle est elle-même substituée par un radical hydroxy, mercapto, carboxy ou anilino], ou un radical alcényle contenant 3 ou 4 atomes de carbone, ou un radical alcynyle contenant 3 ou 4 atomes de carbone, ou bien $R_1$ et $R_2$ forment ensemble avec l'atome d'azote auquel ils sont liés un hétérocycle à 5 ou 6 chaînons contenant éventuellement un autre hétéroatome choisi parmi l'oxygène, le soufre ou l'azote (éventuellement substitué par un radical alcoyle), ou bien lorsque Y est défini comme précédemment et les autres symboles sont définis comme précédemment en b), caractérisé en ce que l'on fait réagir un produit de formule générale :

R'''—H

dans laquelle R''' a la définition de R donnée en b) sur un produit de formule générale :

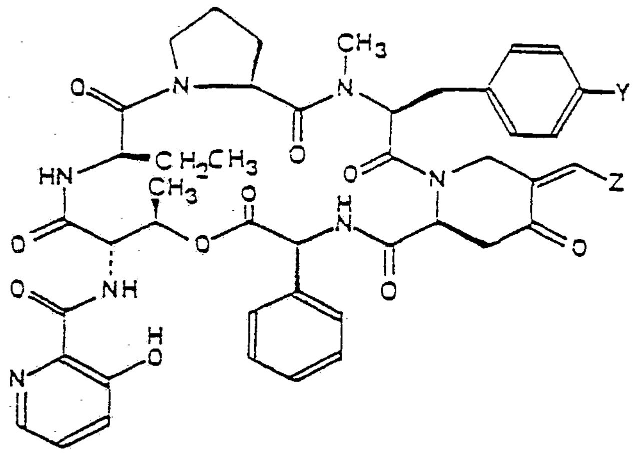

dans laquelle Y est défini comme précédemment, 1 et Z représente un atome d'halogène, un radical triméthylsilyloxy, dialcoyloxyphosphoryloxy ou un radical de formule générale :

$$—OSO_2R_3$$

ou

$$—OCOR_4$$

dans lesquelles $R_3$ est un radical alcoyle, trifluorométhyle, trichlorométhyle ou un radical phényle éventuellement substitué par un atome d'halogène ou par un radical alcoyle ou nitro et $R_4$ est défini comme $R_3$ ou représente un radical alcoylcarbonylméthyle, alcoylcarbonyléthyle, alcoyloxycarbonylméthyle, alcoyloxycarbonyl-2 éthyle ou alcoyloxy, puis lorsqu'ils existent sépare éventuellement les isomères du produit obtenu, et transforme éventuellement le produit obtenu en un sel pharmaceutiquement acceptable.

**Claims** (for the Contracting States : BE, CH, DE, FR, GB, IT, LI, LU, NL, SE)

1. New synergistin derivative, characterized in that it corresponds to the general formula :

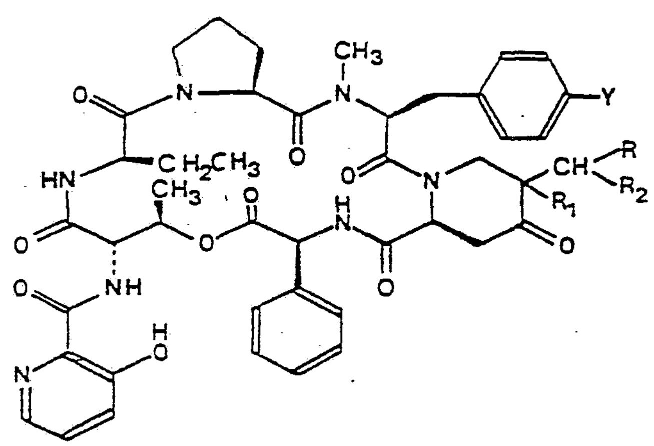

in which Y denotes a hydrogen atom or a dimethylamino radical and

a) either $R_1$ and $R_2$ each denote a hydrogen atom and R denotes a 3-pyrrolidinylthio or 3- or 4-piperidylthio radical (these radicals optionally being substituted with an alkyl radical) or alternatively R denotes an alkylthio radical substituted with one or two hydroxysulphonyl, alkylamino or dialkylamino (optionally substituted with a mercapto or dialkylamino radical) radicals or with one or two rings chosen from piperazino (optionally substituted with an alkyl or mercaptoalkyl radical), morpholino, thiomorpholino, piperidino, 1-pyrrolidinyl, 2-, 3- or 4-piperidyl and 2- or 3-pyrrolidinyl (these latter two rings optionally being substituted on the nitrogen atom with an alkyl radical),

b) or $R_1$ and $R_2$ together form a valency bond and R denotes a 3-pyrrolidinylamino, 3- or 4-piperidylamino, 3-pyrrolidinyloxy, 3- or 4-piperidyloxy, 3-pyrrolidinylthio, or 3- or 4-piperidylthio radical (these radicals optionally being substituted on the nitrogen atom of the ring with an alkyl radical), or

alternatively R denotes an alkylamino, alkyloxy or alkylthio radical substituted with one or two hydroxysulphonyl, alkylamino or dialkylamino (optionally substituted with a dialkylamino radical), trialkylammonio or 4- or 5-imidazolyl radicals or with one or two rings chosen from piperazino (optionally substituted with an alkyl or mercaptoalkyl radical), morpholino, thiomorpholino, piperidino, 1-pyrrolidinyl, 2-, 3- or 4-piperidyl and 2- or 3-pyrrolidinyl (these latter two rings optionally being substituted on the nitrogen atom with an alkyl radical), with the proviso that the alkyl radicals and alkyl portions contain 1 to 5 carbon atoms in a straight or branched chain, in its isomeric forms where these exist and the mixtures thereof, as well as the pharmaceutically acceptable salts thereof.

2. Process for preparing a product according to Claim 1 in the formula of which product Y is defined as in Claim 1 and the other symbols are defined as in Claim 1 at a), characterized in that a product of general formula :

R'—H

in which R' has the definition of R given at a) in Claim 1, is reacted with a product of general formula :

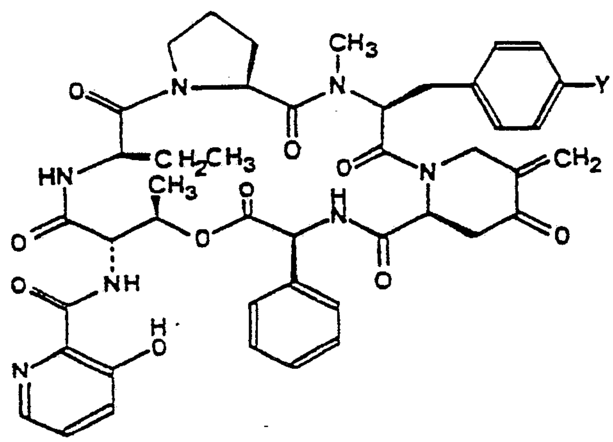

in which Y is defined as in Claim 1, and the product is then isolated and optionally converted to a pharmaceutically acceptable salt.

3. Process for preparing a product according to Claim 1 in the formula of which product Y is defined as in Claim 1 and the other symbols are defined as in Claim 1 at b), with the exception of R denoting a 3-pyrrolidinyloxy, 3- or 4-piperidyloxy or alkyloxy radical (optionally substituted as defined at b) in Claim 1), characterized in that a product of general formula :

R"—H

in which R" has the definition of R given in Claim 1 at b) with the exception of denoting a 3-pyrrolidinyloxy, 3- or 4-piperidyloxy or alkyloxy radical (optionally substituted as defined at b) in Claim 1), is reacted with a product of general formula :

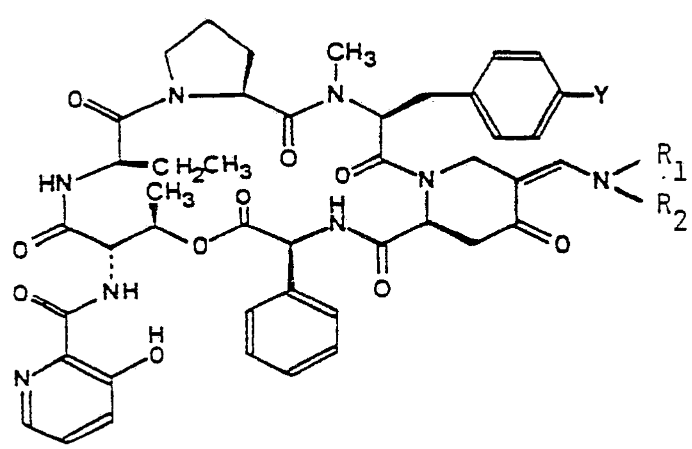

in which Y is defined as in Claim 1 and $R_1$ and $R_2$, which may be identical or different, denote a hydrogen atom or a phenyl or pyridyl radical which is optionally substituted (with a dialkylamino radical in which the

alkyl portion contains 1 to 4 carbon atoms in a straight or branched chain) or an alkyl radical containing 1 to 10 carbon atoms in a straight or branched chain and which is optionally substituted (with a hydroxy, mercapto, carboxy, pyridyl, anilino or alkylamino radical or a dialkylamino radical in which at least one of the alkyl portions it itself substituted with a hydroxy, mercapto, carboxy or anilino radical), or an alkenyl radical containing 3 or 4 carbon atoms, or an alkynyl radical containing 3 or 4 carbon atoms, or alternatively $R_1$ and $R_2$ form, together with the nitrogen atom to which they are linked, a 5- or 6-membered heterocyclic system optionally containing another hetero atom chosen from oxygen, sulphur or nitrogen (and optionally substituted with an alkyl radical), the isomers of the product obtained, where these exist, are then optionally separated and the product obtained is optionally converted to a pharmaceutically acceptable salt.

4. Process for preparing a product according to Claim 1 in the formula of which product Y is defined as in Claim 1 and the other symbols are defined as in Claim 1 at b), characterized in that a product of general formula :

$$R'''—H$$

in which R''' has the definition of R given at b) in Claim 1, is reacted with a product of general formula :

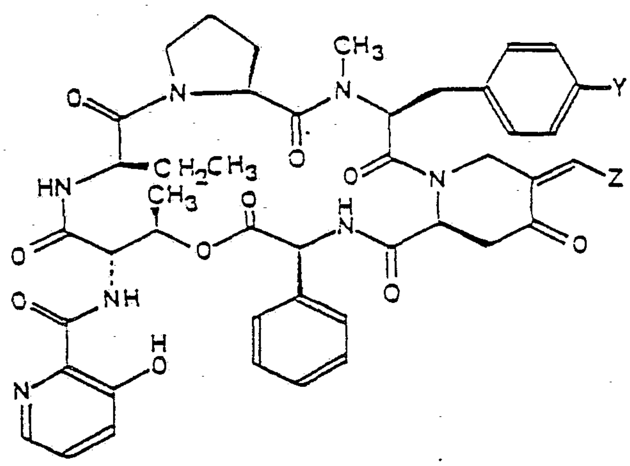

in which Y is defined as in Claim 1 and Z denotes a halogen atom, a trimethylsilyloxy or dialkyloxyphosphoryloxy radical or a radical of general formula

$$—OSO_2R_3$$

or

$$—OCOR_4$$

in which formulae $R_3$ is an alkyl, trifluoromethyl or trichloromethyl radical or a phenyl radical optionally substituted with a halogen atom or with an alkyl or nitro radical and $R_4$ is defined as $R_3$ or denotes an alkylcarbonylmethyl, alkylcarbonylethyl, alkyloxycarbonylmethyl, 2-(alkyloxycarbonyl)ethyl or alkyloxy radical, the isomers of the product obtained, where these exist, are then optionally separated and the product obtained is optionally converted to a pharmaceutically acceptable salt.

5. Pharmaceutical composition, characterized in that it contains at least one derivative according to Claim 1, in combination with one or more diluents or adjuvants which are compatible and pharmaceutically acceptable.

**Claim** (for the Contracting State AT)

A process for preparing new synergistin derivatives of general formula :

(See formula page 49)

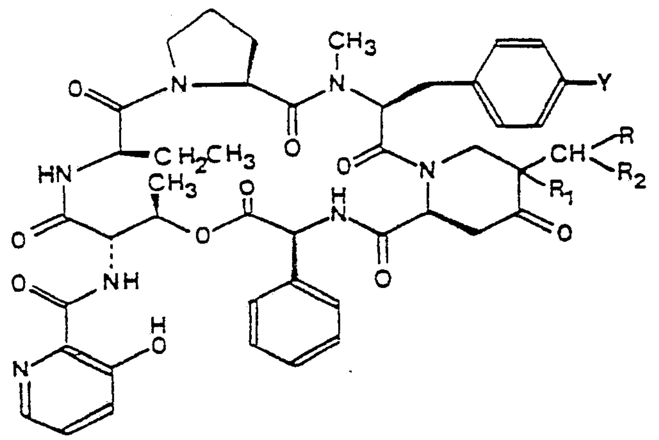

(I)

in which Y denotes a hydrogen atom or a dimethylamino radical and

a) either $R_1$ and $R_2$ each denote a hydrogen atom and R denotes a 3-pyrrolidinylthio or 3- or 4-piperidylthio radical (these radicals optionally being substituted with an alkyl radical) or alternatively R denotes an alkylthio radical substituted with one or two hydroxysulphonyl, alkylamino or dialkylamino (optionally substituted with a mercapto or dialkylamino radical) radicals or with one or two rings chosen from piperazino (optionally substituted with an alkyl or mercaptoalkyl radical), morpholino, thiomorpholino, piperidino, 1-pyrrolidinyl, 2-, 3- or 4-piperidyl and 2- or 3-pyrrolidinyl (these latter two rings optionally being substituted on the nitrogen atom with an alkyl radical),

b) or $R_1$ and $R_2$ together form a valency bond and R denotes a 3-pyrrolidinylamino, 3- or 4-piperidylamino, 3-pyrrolidinyloxy, 3- or 4-piperidyloxy, 3-pyrrolidinylthio, or 3- or 4-piperidylthio radical (these radicals optionally being substituted on the nitrogen atom of the ring with an alkyl radical), or alternatively R denotes an alkylamino, alkyloxy or alkylthio radical substituted with one or two hydroxysulphonyl, alkylamino or dialkylamino (optionally substituted with a dialkylamino radical), trialkylammonio or 4- or 5-imidazolyl radicals or with one or two rings chosen from piperazino (optionally substituted with an alkyl or mercaptoalkyl radical), morpholino, thiomorpholino, piperidino, 1-pyrrolidinyl, 2-, 3- or 4-piperidyl and 2- or 3-pyrrolidinyl (these latter two rings optionally being substituted on the nitrogen atom with an alkyl radical), with the proviso that the alkyl radicals and alkyl portions contain 1 to 5 carbon atoms in a straight or branched chain, in its isomeric forms where these exist and the mixtures thereof, as well as the pharmaceutically acceptable salts thereof, characterized in that, when Y is defined as above and the other symbols are defined as above at a), a product of general formula :

$$R'—H$$

in which R′ has the definition of R given above at a), is reacted with a product of general formula :

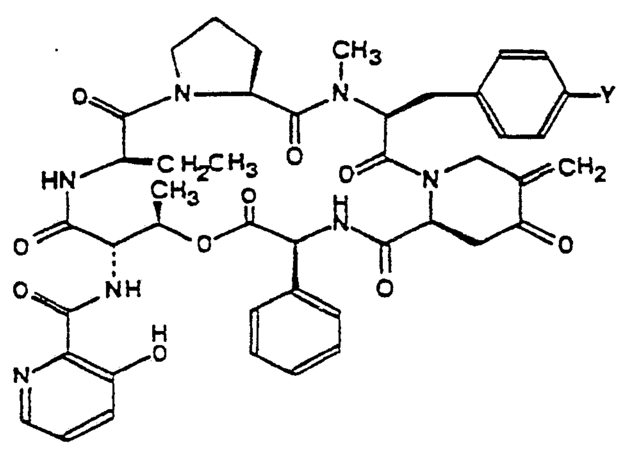

in which Y is defined as above, or alternatively, when Y is defined as above and the other symbols are defined as above at b), with the exception of R denoting a 3-pyrrolidinyloxy, 3- or 4-piperidyloxy or alkyloxy radical (optionally substituted as defined above at b), characterized in that a product of general formula :

$$R''—H$$

in which R″ has the definition of R given above at b) with the exception of denoting a 3-pyrrolidinyloxy, 3-

or 4-piperidyloxy or alkyloxy radical (optionally substituted as defined at b), is reacted with a product of general formula :

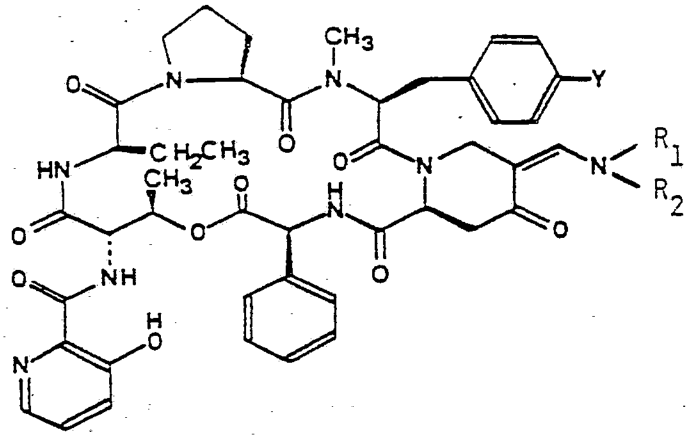

in which Y is defined as above and $R_1$ and $R_2$, which may be identical or different, denote a hydrogen atom or a phenyl or pyridyl radical which is optionally substituted (with a dialkylamino radical in which the alkyl portion contains 1 to 4 carbon atoms in a straight or branched chain) or an alkyl radical containing 1 to 10 carbon atoms in a straight or branched chain and optionally substituted (with a hydroxy, mercapto, carboxy, pyridyl, anilino or alkylamino radical or a dialkylamino radical in which at least one of the alkyl portions is itself substituted with a hydroxy, mercapto, carboxy or anilino radical), or an alkenyl radical containing 3 or 4 carbon atoms, or an alkynyl radical containing 3 or 4 carbon atoms, or alternatively $R_1$ and $R_2$ form, together with the nitrogen atom to which they are linked, a 5- or 6-membered heterocyclic system optionally containing another heteroatom chosen from oxygen, sulphur or nitrogen (and optionally substituted with an alkyl radical), or alternatively, when Y is defined as above and the other symbols are defined as above at b), characterized in that a product of general formula :

$$R'''—H$$

in which R''' has the definition R given at b), is reacted with a product of general formula :

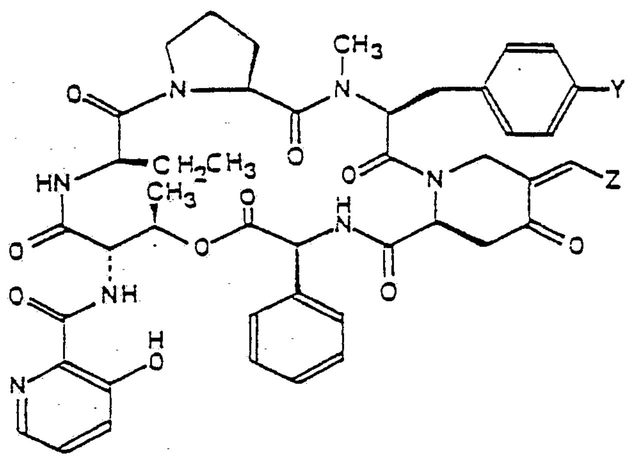

in which Y is defined as above and Z denotes a halogen atom, a trimethylsilyloxy or dialkyloxyphosphoryloxy radical or a radical of general formula

$$—OSO_2R_3$$

or

$$—OCOR_4$$

in which formulae $R_3$ is an alkyl, trifluoromethyl or trichloromethyl radical or a phenyl radical optionally substituted with a halogen atom or with an alkyl or nitro radical and $R_4$ is defined as $R_3$ or denotes an alkylcarbonylmethyl, alkylcarbonylethyl, alkyloxycarbonylmethyl, 2-(alkyloxycarbonyl)ethyl or alkyloxy radical, the isomers of the product obtained, where these exist, are then optionally separated and the product obtained is optionally converted to a pharmaceutically acceptable salt.

**Patentansprüche** (für die Vertragsstaaten : BE, CH, DE, FR, GB, IT, LI, LU, NL, SE)

1. Neues Derivat von Synergistinen, dadurch gekennzeichnet, daß es der allgemeinen Formel

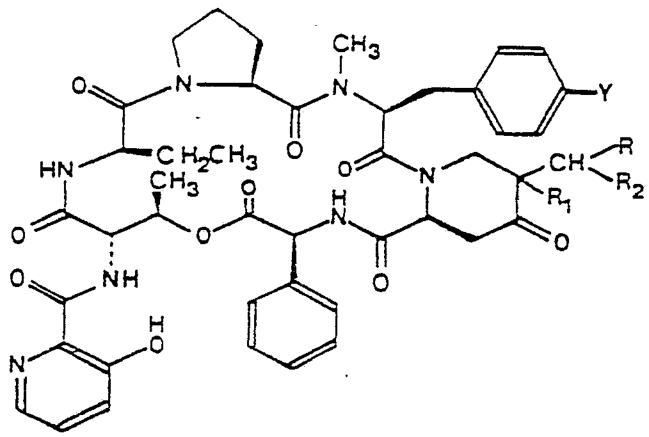

entspricht, worin Y ein Wasserstoffatom oder einen Dimethylaminorest bedeutet, und

a) entweder $R_1$ und $R_2$ jeweils ein Wasserstoffatom bedeuten, und R einen Pyrrolidinyl-3-thio- oder Piperidyl-3- oder -4-thiorest bedeutet (wobei diese Reste gegebenenfalls durch einen Alkylrest substituiert sind), oder aber R bedeutet einen Alkylthiorest, substituiert durch ein oder zwei Hydroxysulfonyl-, Alkylamino-, Dialkylaminoreste (gegebenenfalls substituiert durch einen Mercapto- oder Dialkylaminorest) oder durch ein oder zwei Cyclen, ausgewählt unter Piperazino (gegebenenfalls substituiert durch einen Alkyl- oder Mercaptoalkylrest), Morpholino, Thiomorpholino, Piperidino, Pyrrolidinyl-1, Piperidyl-2, -3 oder -4 und Pyrrolidinyl-2 oder -3 (wobei diese beiden letzteren Cyclen gegebenenfalls am Stickstoffatom durch einen Alkylrest substituiert sind),

b) oder $R_1$ und $R_2$ bilden zusammen eine Valenzbindung, und R bedeutet einen Rest Pyrrolidinyl-3-amino, Piperidyl-3- oder -4-amino, Pyrrolidinyl-3-oxy, Piperidyl-3- oder -4-oxy, Pyrrolidinyl-3-thio, Piperidyl-3- oder -4-thio (wobei diese Reste gegebenenfalls am Stickstoffatom des Ringes durch einen Alkylrest substituiert sind), oder aber R bedeutet einen Alkylamino-, Alkyloxy- oder Alkylthiorest, substituiert durch ein oder zwei Reste Hydroxysulfonyl, Akylamino, Dialkylamino (gegebenenfalls substituiert durch einen Dialkylaminorest), Trialkylammonio oder Imidazolyl-4 oder -5 oder durch ein oder zwei Cyclen, ausgewählt unter Piperazino (gegebenenfalls substituiert durch einen Alkyl- oder Mercaptoalkylrest), Morpholino, Thiomorpholino, Piperidino, Pyrrolidinyl-1, Piperidyl-2, -3 oder -4 und Pyrrolidinyl-2 oder -3 (wobei die beiden letzteren Ringe gegebenenfalls am Stickstoffatom durch einen Alkylrest substituiert sind), wobei die Alkylreste und Alkylteile 1 bis 5 Kohlenstoffatome in gerader oder verzweigter Kette enthalten, unter seinen isomeren Formen, wenn sie existieren, und ihren Mischungen sowie ihre pharmazeutisch annehmbaren Salze.

2. Verfahren zur Herstellung eines Produkts gemäß Anspruch 1, in dessen Formel Y wie in Anspruch 1 definiert ist, und die übrigen Symbole wie in Anspruch 1 in a) definiert sind, dadurch gekennzeichnet, daß man ein Produkt der allgemeinen Formel

R'—H

worin R' die für R in a) in Anspruch 1 angegebene Definition hat, auf ein Produkt der allgemeinen Formel

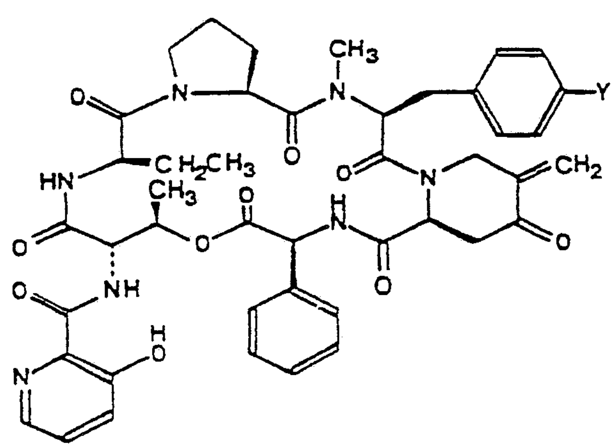

worin Y wie in Anspruch 1 definiert ist, einwirken läßt, das Produkt dann isoliert und es gegebenenfalls in ein pharmazeutisch annehmbares Salz überführt.

3. Verfahren zur Herstellung eines Produkts gemäß Anspruch 1, in dessen Formel Y wie in Anspruch 1 definiert ist und die übrigen Symbole wie in Anspruch 1 in b) definiert sind, mit der Ausnahme, daß R einen Pyrrolidinyl-3-oxy-, Piperidyl-3- oder -4-oxy- oder Alkyloxyrest (gegebenenfalls substituiert wie unter b) in Anspruch 1 definiert), bedeutet, dadurch gekennzeichnet, daß man ein Produkt der allgemeinen Formel

R″—H

worin R″ die Definition von R, die in Anspruch 1 unter b) angegeben ist, hat, mit Ausnahme der Bedeutung eines Restes Pyrrolidinyl-3-oxy, Piperidyl-3 oder -4-oxy oder Alkyloxy (gegebenenfalls substituiert wie unter b) in Anspruch 1 definiert), auf ein Produkt der allgemeinen Formel

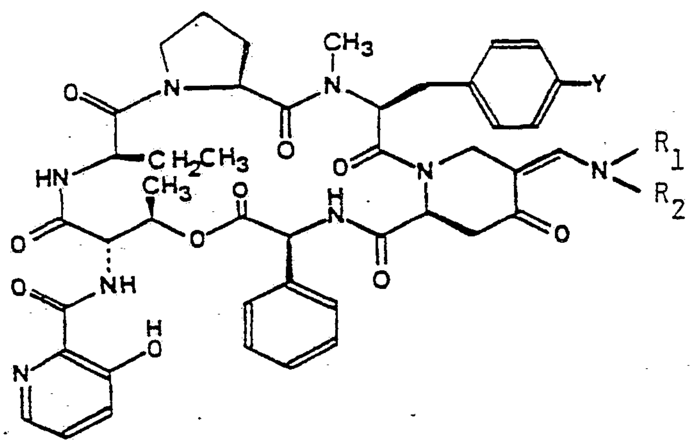

einwirken läßt, worin Y wie in Anspruch 1 definiert ist und $R_1$ und $R_2$, die identisch oder verschieden sind, ein Wasserstoffatom oder einen Phenyl- oder Pyridylrest, gegebenenfalls substituiert (durch einen Dialkylaminorest, dessen Alkylteil 1 bis 4 Kohlenstoffatome in gerader oder verzweigter Kette enthält) oder einen Alkylrest mit 1 bis 10 Kohlenstoffatomen in gerader oder verzweigter Kette, gegebenenfalls substituiert (durch einen Rest Hydroxy, Mercapto, Carboxy, Pyridyl, Anilino, Alkylamino oder Dialkylamino, wovon wenigstens einer der Alkylteile seinerseits durch einen Hydroxy-, Mercapto-, Carboxy- oder Anilinorest substituiert ist) oder einen Alkenylrest mit 3 oder 4 Kohlenstoffatomen oder einen Alkinylrest mit 3 oder 4 Kohlenstoffatomen bedeutet, oder aber $R_1$ und $R_2$ bilden zusammen mit dem Stickstoffatom, an das sie gebunden sind, einen Heterocyclus mit 5 oder 6 Kettengliedern, enthaltend gegebenenfalls ein weiteres Heteroatom, ausgewählt unter Sauerstoff, Schwefel oder Stickstoff (gegebenenfalls substituiert durch einen Alkylrest), daß man dann, falls sie existieren, gegebenenfalls die Isomeren des erhaltenen Produkts trennt, und gegebenenfalls das erhaltene Produkt in ein pharmazeutisch annehmbares Salz überführt.

4. Verfahren zur Herstellung eines Produkts gemäß Anspruch, in dessen Formel Y wie in Anspruch 1 definiert ist und die übrigen Symbole wie in Anspruch 1 unter b) definiert sind, dadurch gekennzeichnet, daß man ein Produkt der allgemeinen Formel

R‴—H

worin R‴ die Definition für R, die in b) in Anspruch 1 angegeben ist, hat, auf ein Produkt der allgemeinen Formel

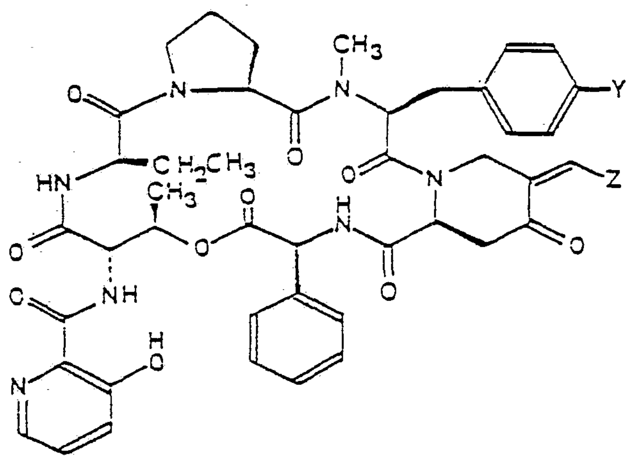

einwirken läßt, worin Y wie in Anspruch 1 definiert ist, und Z ein Halogenatom, einen Trimethylsilyloxy-, Dialkyloxyphosphoryloxyrest oder einen Rest der allgemeinen Formel

$$—OSO_2R_3$$

oder

$$—OCOR_4$$

bedeutet, worin $R_3$ ein Alkyl-, Trifluormethyl-, Trichlormethylrest oder ein Phenylrest ist, der gegebenenfalls durch ein Halogenatom oder durch einen Alkyl- oder Nitrorest substituiert ist, und $R_4$ wie für $R_3$ definiert ist, oder einen Alkylcarbonylmethyl-, Alkylcarbonylethyl-, Alkyloxycarbonylmethyl-, 2-Alkyloxycarbonyl-ethyl- oder Alkyloxyrest bedeutet, daß man dann, falls sie existieren, gegebenenfalls die Isomeren des erhaltenen Produkts trennt und das erhaltene Produkt gegebenenfalls in ein pharmazeutisch annehmbares Salz überführt.

5. Pharmazeutische Zusammensetzung, dadurch gekennzeichnet, daß sie mindestens ein Derivat gemäß Anspruch 1 zusammen mit einem oder mehreren verträglichen und pharmazeutisch annehmbaren Verdünnungs- oder Hilfsmitteln enthält.

**Patentanspruch** (für den Vertragsstaat AT)

Verfahren zur Herstellung neuer Synergistinderivate der allgemeinen Formel :

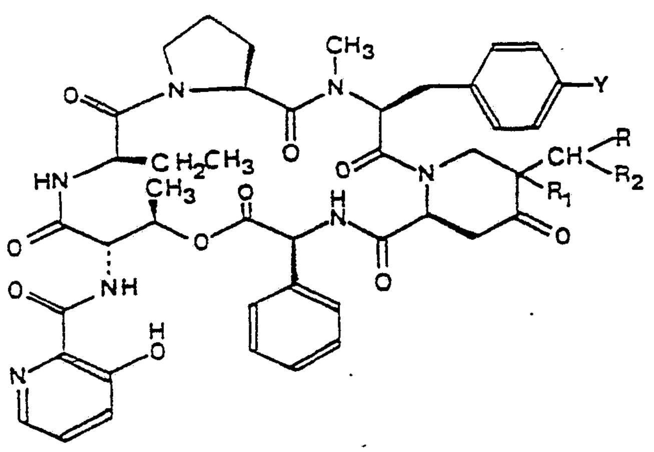

(I)

in welcher Y ein Wasserstoffatom oder einen Dimethylaminorest darstellt und

a) entweder stellen $R_1$ und $R_2$ jeweils ein Wasserstoffatom dar und R einen 3-Pyrrolidinyl-thio- oder 3- oder 4-Piperidyl-thiorest dar (diese Reste sind gegebenenfalls durch eine Alkylgruppe substituiert) oder R stellt einen Alkylthiorest, substituiert durch einen oder zwei Hydroxysulfonyl-, Alkylamino-, Dialkylamino- (gegebenenfalls substituiert durch einen Mercapto- oder Dialkylaminorest) oder durch einen oder zwei Ringe, ausgewählt aus Piperazino (gegebenenfalls substituiert durch einen Alkyl- oder Mercaptoalkylrest), Morpholino, Thiomorpholino, Piperidino, 1-Pyrrolidinyl-, 2-, 3- oder 4-Piperidyl- und 2- oder 3-Pyrrolidinyl- (die beiden letzteren Ringe sind gegebenenfalls am Stickstoffatom substituiert durch einen Alkylrest) dar

b) oder $R_1$ und $R_2$ bilden zusammen eine Valenzbindung und R stellt einen 3-Pyrrolidinylamino-, 3- oder 4-Piperidylamino-, 3-Pyrrolidinyloxy-, 3- oder 4-Piperidyloxy-, 3-Pyrrolidinyl-thio-, 3- oder 4-Piperidyl-thiorest (diese Reste können gegebenenfalls am Stickstoffatom des Ringes durch einen Alkylrest substituiert sein) dar oder R stellt einen Alkylamino-, Alkyloxy- oder Alkylthiorest, substituiert durch einen oder zwei Hydroxysulfonyl-, Alkylamino-, Dialkylamino- (gegebenenfalls substituiert durch einen Dialkylaminorest), Trialkylammonium- oder 4- oder 5-Imidazolylreste oder durch einen oder zwei Ringe, ausgewählt aus Piperazino (gegebenenfalls substituiert durch einen Alkyl- oder Mercaptoalkylrest), Morpholino, Thiomorpholino, Piperidino-, 1-Pyrrolidinyl-, 2-, 3- oder 4-Piperidyl und 2- oder 3-Pyrrolidinyl (die beiden letzten Ringe sind gegebenenfalls am Stickstoffatom substituiert durch einen Alkylrest), dar, wobei die Alkylreste und Alkylteile selbstverständlich 1 bis 5 Kohlenstoffatome in gerader oder verzweigter Kette enthalten, sowie von ihren pharmazeutisch zulässigen Salzen, dadurch gekennzeichnet, daß man wenn Y die obige Bedeutung hat und die übrigen Symbole die oben unter a) angegebene Bedeutung haben, eine Verbindung der allgemeinen Formel :

$$R'—H,$$

in welcher R' die unter a) angegebene Bedeutung von R hat, auf eine Verbindung der allgemeinen Formel :

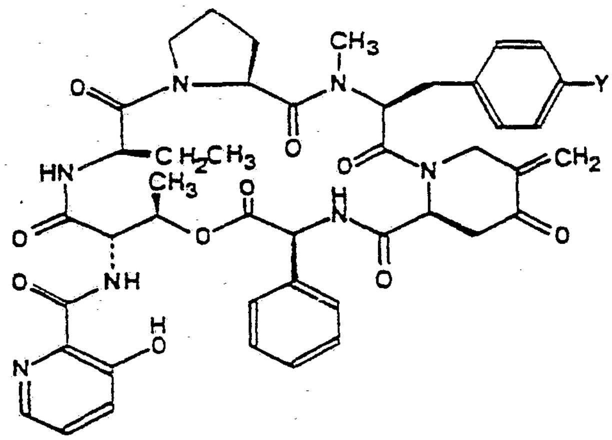

in welcher Y die obige Bedeutung hat, einwirken läßt, oder wenn Y die obige Bedeutung hat und die übrigen Symbole die oben unter b) angegebene Bedeutung haben, mit Ausnahme eines 3-Pyrrolidinyloxy-, 3- oder 4-Piperidyloxy- oder Alkyloxyrestes (gegebenenfalls substituiert wie oben unter b) angegeben) für R, dadurch gekennzeichnet, daß man eine Verbindung der allgemeinen Formel :

R"—H,

in welcher R" die unter b) angegebene Bedeutung von R hat, ausgenommen einen 3-Pyrrolidinyloxy-, 3- oder 4-Piperidyloxy- oder Alkyloxyrest (gegebenenfalls wie unter b) angegeben substituiert) mit einer Verbindung der allgemeinen Formel :

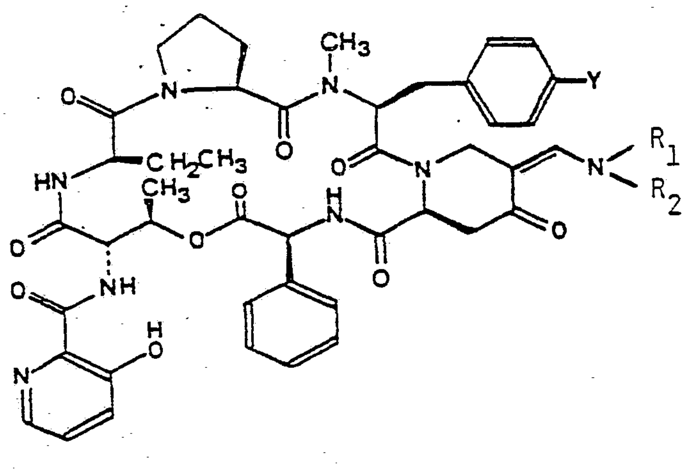

in welcher Y die obige Bedeutung hat und $R_1$ und $R_2$ unabhängig voneinander ein Wasserstoffatom oder einen Phenyl- oder Pyridylrest, gegebenenfalls substituiert (durch einen Dialkylaminorest, dessen Alkylteil 1 bis 4 Kohlenstoffatome in gerader oder verzweigter Kette enthält) oder einen Alkylrest mit 1 bis 10 Kohlenstoffatomen in gerader oder verzweigter Kette, gegebenenfalls substituiert (durch einen Hydroxy-, Mercapto-, Carboxy-, Pyridyl-, Anilino-, Alkylamino- oder Dialkylaminorest, worin zumindest einer der Alkylteile seinerseits durch einen Hydroxy-, Mercapto-, Carboxy- oder Anilinorest substituiert ist), oder einen Alkenylrest mit 3 oder 4 Kohlenstoffatomen oder einen Alkynylrest mit 3 oder 4 Kohlenstoffatomen darstellen oder $R_1$ und $R_2$ zusammen mit dem Stickstoffatom, an das sie gebunden sind, einen Heterocyclus mit 5 oder 6 Gliedern bilden, der gegebenenfalls ein weiteres Hereroatom, ausgewählt aus Sauerstoff, Schwefel oder Stickstoff (gegebenenfalls substituiert durch einen Alkylrest) enthält, oder wenn Y die obige Bedeutung hat und die übrigen Symbole die unter b) angegebene Bedeutung haben, dadurch gekennzeichnet, daß man eine Verbindung der allgemeinen Formel :

R'''—H

in welcher R''' die unter b) angegebene Bedeutung von R hat, mit einer Verbindung der allgemeinen Formel :

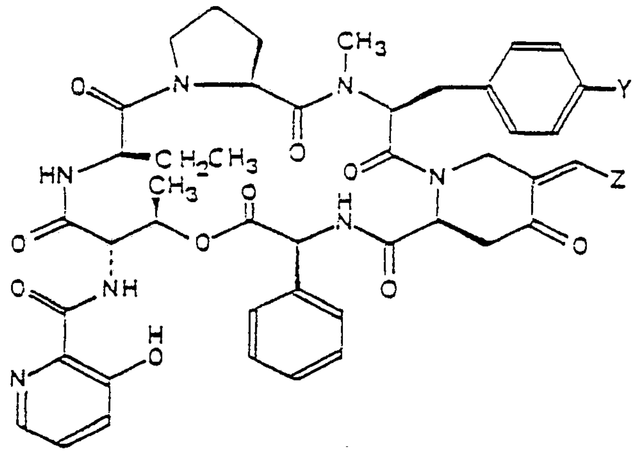

in welcher Y die obige Bedeutung hat und Z ein Halogenatom, einen Trimethylsilyloxy-, Dialkyloxyphosphoryloxyrest oder einen Rest der allgemeinen Formel

$$-OSO_2R_3$$

oder

$$-OCOR_4$$

darstellt, in welchen $R_3$ ein Alkyl-, Trifluormethyl-, Trichlormethyl- oder gegebenenfalls durch ein Halogenatom oder durch einen Alkyl- oder Nitrorest substituierter Phenylrest ist und $R_4$ die Bedeutung von $R_3$ hat oder einen Alkylcarbonylmethyl-, Alkylcarbonyläthyl-, Alkyloxycarbonylmethyl-, 2-Alkyloxycarbonyläthyl- oder Alkyloxyrest darstellt, reagieren läßt und daß man dann, falls existent, gegebenenfalls die Isomeren der erhaltenen Verbindung trennt und gegebenenfalls die erhaltene Verbindung in ein pharmazeutisch zulässiges Salz überführt.